(19) Europäisches Patentamt / European Patent Office / Office européen des brevets

(11) **EP 4 786 479 A1**

(12) **EUROPEAN PATENT APPLICATION**
published in accordance with Art. 153(4) EPC

(43) Date of publication:
**05.08.2026 Bulletin 2026/32**

(21) Application number: **24873066.5**

(22) Date of filing: **27.09.2024**

(51) International Patent Classification (IPC):
***C07H 21/02*** (2006.01) ***C07H 1/04*** (2006.01)
***A61K 39/00*** (2006.01) ***A61K 48/00*** (2006.01)
***C12N 15/63*** (2006.01)

(52) Cooperative Patent Classification (CPC):
**A61K 39/00; A61K 48/00; C07H 1/04; C07H 21/02; C12N 15/63**

(86) International application number:
**PCT/KR2024/014788**

(87) International publication number:
**WO 2025/071359 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 KR 20230130741**

(71) Applicant: **Hanmi Fine Chemical Co., Ltd.**
**Siheung-Si, Gyeonggi-do 15093 (KR)**

(72) Inventors:
- **LEE, Jae Koo**
  **Siheung-si, Gyeonggi-do 15093 (KR)**
- **KIM, Ji Hye**
  **Siheung-si, Gyeonggi-do 15093 (KR)**
- **JANG, Min Gyeong**
  **Siheung-si, Gyeonggi-do 15093 (KR)**
- **KIM, Na Ri**
  **Siheung-si, Gyeonggi-do 15093 (KR)**
- **MIN, Su Hyeon**
  **Siheung-si, Gyeonggi-do 15093 (KR)**

(74) Representative: **Santarelli**
**Tour Trinity**
**1 bis Place de la Défense**
**92400 Courbevoie (FR)**

(54) **MRNA CAP ANALOG AND USE THEREOF**

(57) Provided are a compound of chemical formula 1, a cap analog comprising same, mRNA 5'-capped with the cap analog, a method for preparing mRNA by using the cap analog, a use of the cap analog for preparing mRNA, and a pharmaceutical composition for expressing a target peptide or protein, comprising mRNA 5'-capped with the cap analog.

FIG. 1

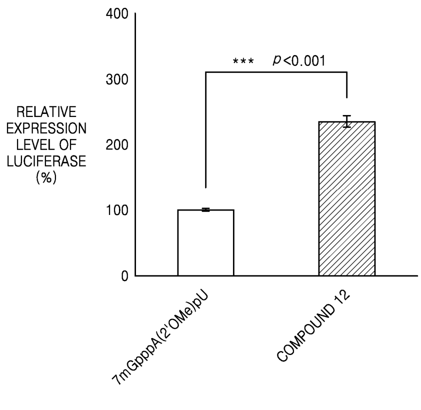


EP 4 786 479 A1

## Description

### Technical Field

The present disclosure relates to a cap analog and a protein expression method using the same, and more particularly, to an mRNA cap analog, a 5'-capped mRNA including the same, a method for preparing the same, a pharmaceutical composition for expressing a target peptide or protein including the 5'-capped mRNA, and a method for producing a target peptide or protein by using the 5'-capped mRNA.

### Background Art

RNA therapeutics have recently been proposed as a new paradigm for treatment of infectious diseases and cancer. Particularly, the development of mRNA vaccines by using in vitro transcription is highlighted as the most promising method for dealing with RNA virus infectious diseases capable of overcoming limits of conventional vaccines such as attenuated, inactivated, and synthetic antigen vaccines. Because RNA virus infectious diseases are relatively highly transmissible and RNA viruses mutate rapidly due to their unstable structures, rapid development of highly safe vaccines therefor is required. mRNA vaccines may shorten the speed of vaccine development because mRNA vaccine candidate materials may be derived within one month in response to these infectious diseases and has a great advantage of excellent safety without the risk of genetic modification, because mRNA introduced into the body is decomposed within 24 hours and, unlike DNA vaccines, is translated into a protein by a ribosome in the cytoplasm without entering the nucleus of a cell.

For in vitro transcription of mRNA molecules, the following components are required: a linearized DNA template, an RNA polymerase (mainly T7 RNA polymerase), four ribonucleoside triphosphates (NTPs) A, G, C, and U (or 1-Methylpseudouridine (m1Ψ)), and a cap analog. If required, poly A polymerase may be used for polyadenylation of 200 to 250 adenosines at the 3' end, and a capping enzyme, instead of a cap analog, may be used to synthesize a 5' cap structure at the 5' end. Compared to other relatively optimized components, the 5' cap structure having a complex structure and playing an important role in protein translation by mRNA molecules accounts for the highest cost, and extensive research has been conducted into efficient synthesis thereof.

mRNA undergoes a maturation process after transcription for effective translation of an encoded protein. In the process, a process of linking 7-methylguanosine ($^{7m}$G) to the 5' end of an mRNA molecule via a 5'- to 5'-triphosphate chain is referred to as a 5' cap process, and the linked structure is referred to as a 5' cap structure ($^{7m}$GpppN). The 5' cap structure may protect the 5' end of mRNA from biodegradation by 5' exonuclease and affects migration of mRNA from the nucleus to the cytoplasm. Particularly, the 5' cap structure is recognized by a eukaryotic translation initiation factor 4E (eIF4E) to form a translation initiation complex, playing an important role in protein expression.

In order for this capped mRNA to be clinically applicable, it must have a structure (Cap1 mRNA) in which the 2'hydroxyl group of the first nucleotide linked to 7-methylguanosine via a 5'-triphosphate chain is methylated. When applying the unmethylated Cap0 structure, MDA5 (an intracellular sensor protein), which recognizes the Cap0 structure, recognizes the 2' hydroxyl group when an exogenous mRNA with a Cap0 structure is introduced into the body and induces an immune-inflammatory response, which impairs the bonding of the mRNA to eIF4E and thus inhibits protein expression. On the contrary, because exogenous Cap1 mRNA, in which the 2'OH is methylated, is not recognized by MDA5 even when introduced into the body, an immune response is not induced and relatively high expression of protein may be induced, and thus Cap1 mRNA is suitable for clinical trials.

Examples of mRNA vaccines commercialized based on the above-described Cap1 mRNA structure are mRNA-1273 of Moderna and BNT162b2 of Pfizer-BioNTech. Both mRNA vaccines were developed to deal with infectious diseases caused by severe acute respiratory syndrome coronavirus type 2 (SARS-CoV-2), an RNA virus that has caused a global pandemic. In the case of the BNT162b2 mRNA vaccine, a co-transcriptional capping method that is a method of synthesizing mRNA by simultaneously introducing a chemically synthesized cap analog during in-vitro mRNA transcription so that the cap analog constitutes the 5' end was adopted, instead of a conventional capping method using a capping enzyme, to reduce manufacturing costs.

A cap analog used in BNT162b2 is Cleancap® AG (3'OMe), as a third-generation cap analog, developed by TriLink Biotechnologies Inc. (US Patent No. 10,913,768). The cap analog is a trinucleotide cap analog having a structure of $^{7m}G_{(3'OMe)}pppA_{(2'OMe)}pG$ and may be introduced during mRNA synthesis to synthesize a Cap1 mRNA structure without using an enzyme. Because the trinucleotide cap analog includes at least one more base than GTP, base pairing hydrogen bonding force of the trinucleotide cap analog in base pairing for initiation of transcription with a strand of a DNA template is stronger than that of GTP. Therefore, unlike a previous second-generation cap analog (US Patent No. 7,074,596) that is a dinucleotide cap analog pairing with a DNA template competitively with guanosine triphosphate (GTP), the trinucleotide cap analog may base pair with the DNA template preferentially over GTP and thus reduce the amount of added cap analog during in vitro mRNA transcription, thereby reducing mRNA impurities having a pppG 5' end.

A shortage of raw materials was once experienced for a while because demand for BNT162b2 including the

trinucleotide cap analog exploded to deal with the global Covid-19 pandemic. Also, the cap analog still accounts for the highest cost in mass production of mRNA vaccines. In order to manufacture mRNA vaccines to appropriately deal with infectious diseases caused by highly transmissible RNA viruses, rapid and economical production and supply of cap analogs are required.

manufacturing process of currently commercialized trinucleotide cap analogs is briefly divided into 6 steps as shown below (US Patent No. 10,913,768).

[Reaction Scheme 1] $G_{(3'OMe)} \rightarrow pG_{(3'OMe)} \rightarrow Im\text{-}pG_{(3'OMe)} \rightarrow ppG_{(3'OMe)} \rightarrow pp^{7m}G_{(3'OMe)} \rightarrow Im\text{-}pp^{7m}G_{(3'OMe)}+pN_{(2'OMe)}\ pN \rightarrow {}^{7m}G_{(3'OMe)}pppN_{(2'OMe)}pN$

In Reaction Scheme 1, the G is guanosine, $^{7m}G$ is 7-methylguanosine, N is one of adenosine, cytidine, guanosine, and uridine, p is $-P(=O)O_2-$, Me is methyl, and Im is imidazolide. Moreover, specifically, a long process of at least 10 steps needs to be performed if a step of synthesizing $pN_{(2'OMe)}pN$, which is a reactant used in a final manufacturing step, is included.

Particularly, a main factor of increasing production costs and synthesis period in the entire manufacturing process for mass production of the TriLink's cap analog is purification steps using ion-exchange chromatography (mainly, DEAE resin) performed between the steps of the manufacturing process. After purifying an intermediate of each process, a large amount of a buffer solution (mainly triethylammonium bicarbonate (TEAB) buffer) used in column purification needs to be distilled to perform a next step in an organic solvent. Because purification is required at each step of producing $pG_{(3'OMe)}$, $ppG_{(3'OMe)}$, $pp^{7m}G_{(3'OMe)}$, $pN_{(2'OMe)}pN$, and $^{7m}G_{(3'OMe)}pppN_{(2'OMe)}pN$ in the entire manufacturing process, a total of 5 ion-exchange column purifications are required. This long process, the column purifications, and the distillation of the buffer solution increase the time and cost for manufacturing the cap analog, thereby lowering economic feasibility.

## Disclosure

### Technical Problem

Provided is a cap analog capable of increasing in vitro synthesis efficiency of 5'-capped mRNA molecules, increasing protein expression efficiency of capped mRNA, and being economically produced by reducing the number of steps of cap analog synthesis and the number of purification processes.

Provided is mRNA 5'-capped with the cap analog.

Provided is a method for preparing mRNA by using the cap analog.

Provided is a composition or kit for preparing 5'-capped mRNA, including the cap analog.

Provided is a pharmaceutical composition for expressing a target peptide or protein, including the cap analog.

Provided is a cell including mRNA 5'-capped with the cap analog.

Provided is a cell including a protein or peptide translated from mRNA 5'-capped with the cap analog.

### Technical Solution

According to an aspect of the present disclosure, a compound of Chemical Formula 1 below or a pharmaceutically acceptable salt thereof is provided:

[Chemical Formula 1]

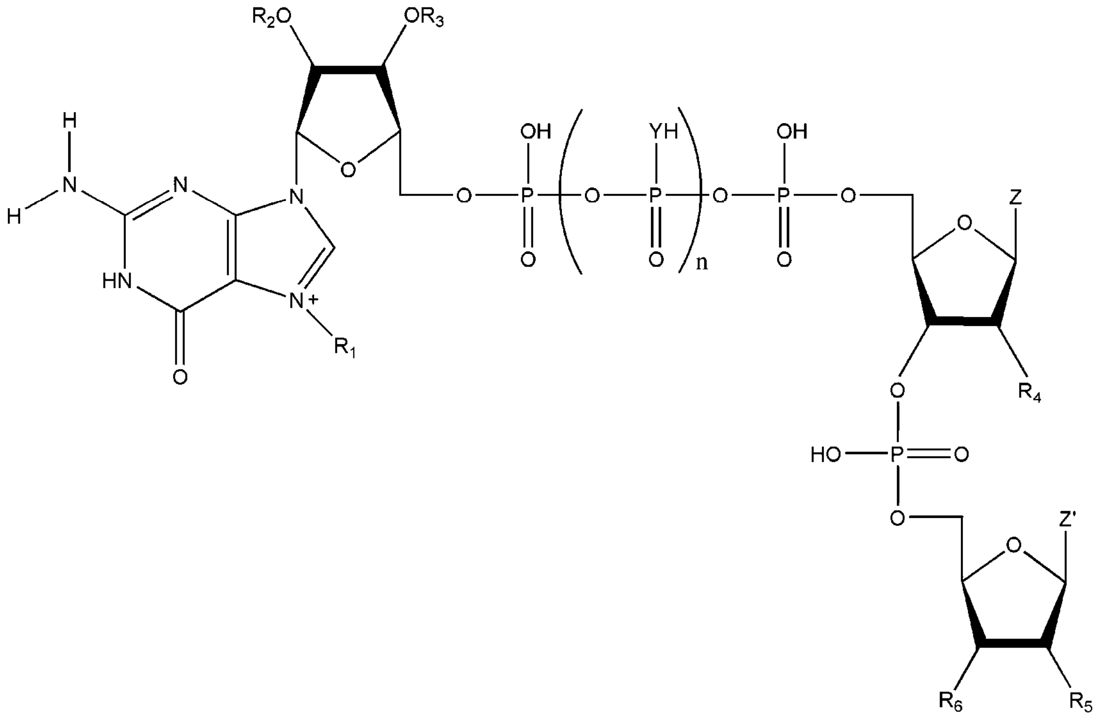

wherein

n = 0, 1, or 2;
YH is OH or SH;
$R_1$ is $C_{1-6}$ alkyl or $CH_2Ph$;
$R_2$ and $R_3$ are each independently H or a sulfonyl-containing group, wherein at least one of $R_2$ and $R_3$ is a sulfonyl-containing group, and the sulfonyl-containing group is independently selected from the group consisting of mesyl, esyl, triflyl, tresyl, tosyl, brosyl, nosyl, and dansyl groups;
$R_4$ and $R_5$ are each independently OH or methoxy;
$R_6$ is OH or a mononucleotide or oligonucleotide having 1 to 7 bases;
Z is each independently a natural, modified, or unnatural nucleoside base; and
Z' is each independently

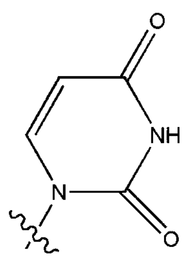 , 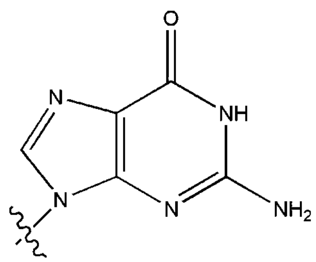 ,

or

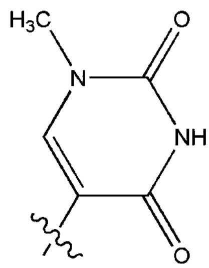 .

Another aspect provides a cap analog that is the compound of Chemical Formula 1.

Another aspect provides mRNA 5'-capped with the cap analog.

Another aspect provides a method for preparing 5'-capped mRNA including adding the cap analog during synthesis of the mRNA.

Another aspect provides a composition or kit for preparing 5'-capped mRNA including the cap analog.

Another aspect provides a pharmaceutical composition for expressing a target peptide or protein including mRNA 5'-capped with the cap analog and a pharmaceutically acceptable carrier.

Another aspect provides a cell including mRNA 5'-capped with the cap analog.

Another aspect provides a cell including a protein or peptide translated from mRNA 5'-capped with the cap analog.

**Advantageous Effects**

The cap analog according to one aspect, like the TriLink's trinucleotide cap analog, may be used in synthesis of mRNA to increase in vitro transcription efficiency of 5'-capped mRNA molecules and increase peptide or protein expression efficiency by capped mRNA. Particularly, because initial protein expression (initial translation) after transfection may further be increased in a cap dependent translation manner, the cap analog may be useful particularly for expression of a gene in which initial protein expression is important (for example, expression of nsp1 in self-amplifying RNA). In addition, the cap analog may be economically synthesized in terms of time and cost by reducing the number of synthesis steps and purifications, the cap analog can produce mRNA with a low content of impurities (e.g., dsRNA, etc.), and the cap analog may contribute to synthesis of 5'-capped mRNA with high economic feasibility together with an increase in expression efficiency of peptide or protein by using the capped mRNA.

Therefore, the cap analog, similar to the currently commercialized trinucleotide cap analog, may increase in vitro transcription efficiency of 5'-capped mRNA molecules and significantly increase peptide or protein expression efficiency by the capped mRNA compared to conventional trinucleotide cap analogs, and may be economically produced.

As such, because the cap analog has excellent advantages in terms of efficacy and production cost, mRNA including the cap analog according to the present disclosure may be very effectively used to treat or prevent diseases of mammals including humans.

**Description of Drawings**

FIG. 1 is a graph showing the relative expression levels of the total luciferase protein translated at 24, 48, and 72 hours after encapsulating luciferase mRNA, capped with the compound (12) of Example 12 according to an embodiment of the present invention or $^{7m}$GpppA$_{(2'OMe)}$pU, into LNPs and transfecting them into HEK293S cell lines.

FIG. 2 is a graph showing the relative expression levels of the total luciferase protein expressed at 3, 6, 9, 24, 48, and 96 hours after encapsulating luciferase mRNA, capped with the compound (12) of Example 12 according to an embodiment of the present invention or $^{7m}$GpppA$_{(2'OMe)}$pU, into LNPs and intravenously injecting them into the tails of mice.

FIG. 3 is a graph showing the results of quantifying the expression levels of the luciferase protein translated at 24, 48, and 72 hours by measuring luciferase activity after encapsulating luciferase mRNA, capped with the compound (16) of Example 16 according to an embodiment of the present invention or $^{m7}$G$_{(3'OMe)}$pppA$_{(2'OMe)}$pG, into LNPs and transfecting them into HEK293S cell lines.

FIG. 4 is a graph showing the results of quantifying the expression levels of the luciferase protein expressed at 3, 6, 9, 24, 48, and 96 hours after encapsulating luciferase mRNA, capped with the compound (16) of Example 16 according to an embodiment of the present invention or $^{m7}$G$_{(3'OMe)}$pppA$_{(2'OMe)}$pG, into LNPs and intravenously injecting them into the tails of mice.

FIG. 5 is a graph showing the results of quantifying the expression levels of the total luciferase protein expressed at 3, 6, 9, 24, 48, and 96 hours after encapsulating luciferase mRNA, capped with the compound (16) of Example 16 according to an embodiment of the present invention or $^{m7}$G$_{(3'OMe)}$pppA$_{(2'OMe)}$pG, into LNPs and intravenously injecting them into the tails of mice.

FIG. 6 is a graph showing the results of quantifying the expression levels of the total luciferase protein translated at 4, 8, 12, 24, and 48 hours after transfecting HEK293T cell lines with luciferase mRNA, capped with the compound (18) of Example 18 according to an embodiment of the present invention or $^{7m}$GpppA$_{(2'OMe)}$pU, using Lipofectamine.

**Mode for Invention**

Hereinafter, the present disclosure will be described in detail.

Unless otherwise defined, all technical terms used herein have the same meaning as commonly understood by one of ordinary skill in the art to which the present disclosure belongs. Although preferred methods or samples are described in the specifications, those similar or equivalent thereto may also be regarded within the scope of the present disclosure. Throughout the specification, numerical values are considered to include the meaning of "about" even if not specified. The contents of all publications disclosed herein are incorporated by reference in their entirety.

Definition of Terms

Terms used herein have the same meaning as defined below, unless otherwise specified.

In the context of a complex of a cap analog and a DNA template, the term, as used herein, "complementary" or "complementarity" refers to standard Watson/Crick base pairing rules. For example, sequence "5'-A-G-T-C-3'" is complementary to sequence "3'-T-C-A-G-5'". Complementarity does not need to be perfect; duplex may contain mismatched base pairs, degenerative, or unmatched nucleotides. Those skilled in the art may determine duplex stability by experimentally considering a number of variables including, for example, length of oligonucleotides, base composition and sequence of oligonucleotides, incidence of mismatched base pairs, ionic strength, components of a hybridization buffer, and reaction conditions.

Complementarity may be "complete" or "entire" where all of the nucleotide bases of two nucleic acid strands are matched according to recognized base pairing rules, it may be "partial" in which only some of the nucleotide bases of a cap analog and a DNA target are matched according to recognized base pairing rules, or it may be "absent" where none of the nucleotide bases of two nucleic acid strands are matched according to recognized base pairing rules.

As used herein, the term "nucleoside" includes all naturally occurring nucleosides. Base rings most commonly found in naturally occurring nucleosides are purine and pyrimidine rings. Naturally occurring purine rings include, for example, adenine, guanine, and N6-methyladenine. Naturally occurring pyrimidine rings include, for example, cytosine, thymine, 5-methylcytosine, and uracil. Naturally occurring nucleosides include, for example, ribo, 2'-O-methyl, or 2'-deoxyribo derivatives of adenosine, guanosine, cytidine, thymidine, uridine, inosine, 7-methylguanosine, or uridine, but are not limited thereto.

As used herein, the terms "nucleoside analog", "modified nucleoside" or "nucleoside derivative" include synthetic nucleosides as described herein. Nucleoside derivatives also include nucleosides having modified base or/and sugar moieties, with or without protecting groups, for example, 2'-deoxy-2'-fluorouridine, 5-fluorouridine, or the like. The compounds and methods provided herein include such base rings and synthetic analogs thereof, as well as unnatural heterocycle-substituted base sugars, and acyclic substituted base sugars. Other nucleoside derivatives that may be used in the present disclosure include, for example, LNA nucleosides, halogen-substituted purines (e.g., 6-fluoropurine), halogen-substituted pyrimidines, N6-ethyladenine, N4-(alkyl)-cytosine, 5-ethylcytosine, and the like (US Patent No. 6,762,298).

The term "base of modified nucleoside" refers to a base of the "modified nucleoside" and includes, for example, halogen-substituted purines (e.g., 6-fluoropurine), halogen-substituted pyrimidines, N6-ethyladenine, N4-(alkyl)-cytosine, and 5-ethylcytosine.

The term "$C_{1-n}$ alkyl" refers to a linear or branched saturated hydrocarbon radical chain including 1 to n carbon atoms. Examples thereof may include methyl, ethyl, n-propyl, isopropyl, n-butyl, isobutyl, t-butyl, n-pentyl, isopentyl, and n-hexyl, but are not limited thereto.

In the process of synthesizing substances, "*In situ* method" refers to performing a next step of a reaction in an original container without additional purification of an obtained product.

An aspect provides a compound of Chemical Formula 1 below or a pharmaceutically acceptable salt thereof:

[Chemical Formula 1]

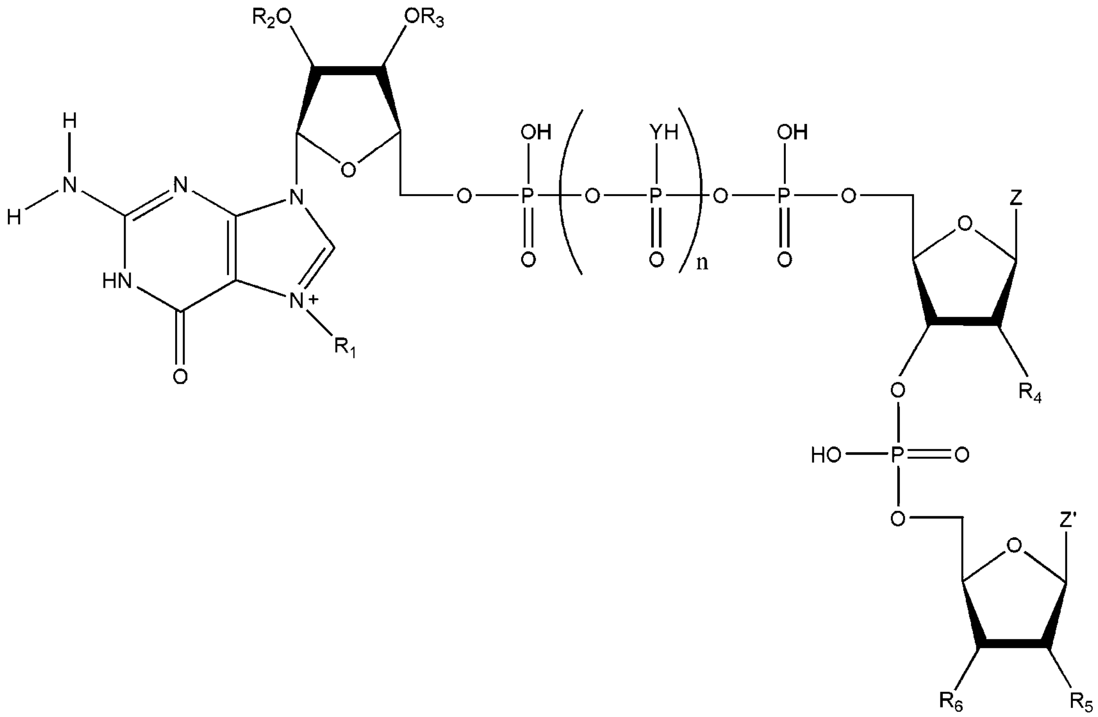

wherein

n = 0, 1, or 2;
YH is OH or SH;
$R_1$ is $C_{1-6}$ alkyl or $CH_2Ph$;
$R_2$ and $R_3$ are each independently H or a sulfonyl-containing group, wherein at least one of $R_2$ and $R_3$ is a sulfonyl-containing group, and the sulfonyl-containing group is independently selected from the group consisting of mesyl, esyl, triflyl, tresyl, tosyl, brosyl, nosyl, and dansyl groups;
$R_4$ and $R_5$ are each independently OH or methoxy;
$R_6$ is OH or a mononucleotide or oligonucleotide having 1 to 7 bases;
Z is each independently a natural, modified, or unnatural nucleoside base; and
Z' is each independently

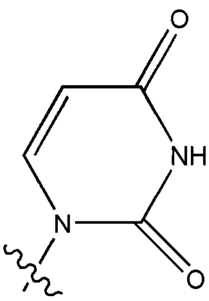
,
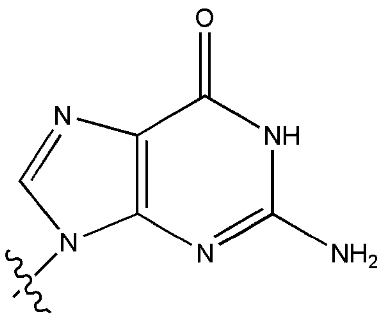
, or
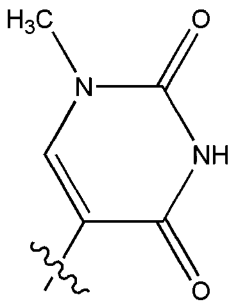
.

The pharmaceutically acceptable salt refers to a salt commonly used in the pharmaceutical field and, specifically, a base addition salt. The salt may be, for example, a monovalent metal salt, a divalent metal salt, an amine salt, or an amino acid salt. The monovalent metal salt may be Na, Li, or K salt, the divalent metal salt may be Ca, Zn, or Mg salt, the amine salt may be trimethylamine, triethylamine, ammonia, pyridine, or picoline salt, and the amino acid salt may be arginine, lysine, or histidine salt, without being limited thereto.
The pharmaceutically acceptable salt may be in the form of a salt that allows the compound of Chemical Formula 1 to form a stable, electrically neutral form in an aqueous liquid.
The compound of Chemical Formula 1 or a pharmaceutically acceptable salt thereof may be a compound of

Chemical Formula 1a below:

[Chemical Formula 1a]

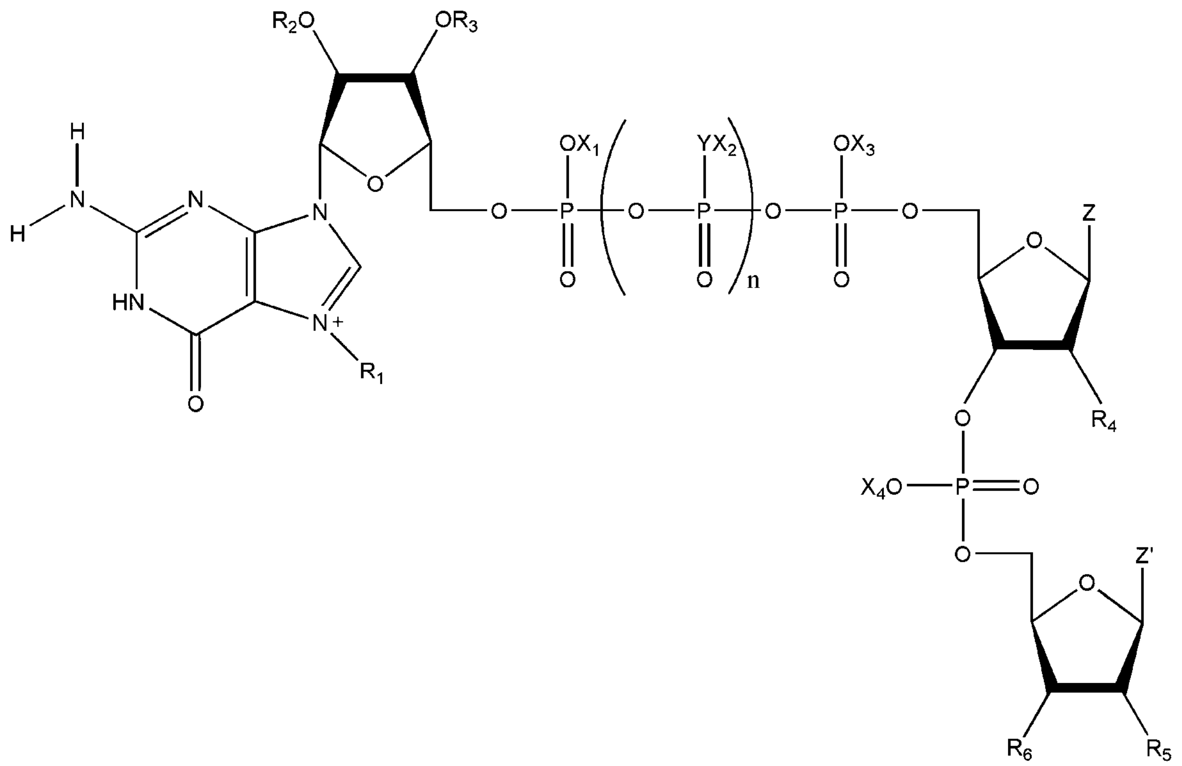

wherein

n = 0, 1, or 2;
$X_1$, $X_2$, $X_{3,}$ and $X_4$ are each independently absent, or a monovalent metal ion, a divalent metal ion, or a combination thereof, wherein $X_1$, $X_2$, $X_{3,}$ and $X_4$ are selected to electrically neutralize the compound of Chemical Formula 1;
Y is O or S;
$R_1$ is $C_{1-6}$ alkyl or $CH_2Ph$;
$R_2$ and $R_3$ are each independently H or a sulfonyl-containing group, wherein at least one of $R_2$ and $R_3$ is a sulfonyl-containing group, and the sulfonyl-containing group is independently selected from the group consisting of mesyl, esyl, triflyl, tresyl, tosyl, brosyl, nosyl, and dansyl groups;
$R_4$ and $R_5$ are each independently OH or methoxy;
$R_6$ is OH or a mononucleotide or oligonucleotide having 1 to 7 bases;
Z is each independently a natural, modified, or unnatural nucleoside base; and
Z' is each independently

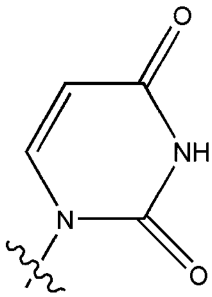, 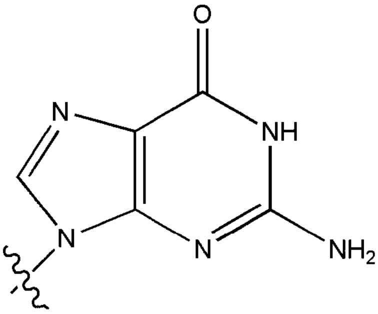, or 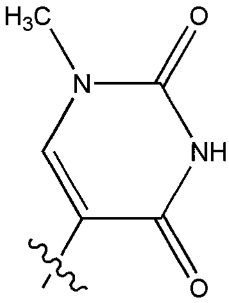.

In an embodiment, the monovalent metal ion is selected from the group consisting of $Na^+$, $Li^+$, and $K^+$, and the divalent metal ion is selected from the group consisting of $Mg^{2+}$, $Zn^{2+}$, and $Ca^{2+}$.
In an embodiment, the sulfonyl-containing group is mesyl, esyl, or tosyl group.
In an embodiment, the $C_{1-6}$ alkyl is methyl, ethyl, propyl, isopropyl, or t-butyl.
In an embodiment, in Chemical Formula 1, Z is a natural purine or pyrimidine base moiety or a modified form thereof. More specifically, the Z may be each independently selected from the group consisting of guanine, adenine, cytosine, thymine, uracil, and modified forms thereof, and in an embodiment, Z is adenine.
In an embodiment, in Chemical Formula 1, $R_6$ is OH, a mononucleotide, or an oligonucleotide having 2 to 5 bases.
In an embodiment, the compound having a structure of Chemical Formula 1a is as follows.
In Chemical Formula 1a,

n = 0, 1, or 2;
$X_1$, $X_2$, $X_3$, and $X_4$ are each independently absent or Na;
Y is O or S;
$R_1$ is $C_{1-6}$ alkyl;
$R_2$ and $R_3$ are each independently H or a sulfonyl-containing group, wherein at least one of $R_2$ and $R_3$ is a sulfonyl-containing group, and the sulfonyl-containing group is independently selected from the group consisting of mesyl, esyl, and tosyl groups;
$R_4$ and $R_5$ are each independently OH or methoxy;
$R_6$ is OH or a mononucleotide or oligonucleotide having 1 to 3 bases; and
Z is a natural, modified, or unnatural nucleoside base; and .
Z' is each independently

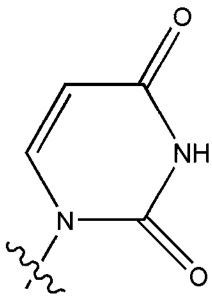, 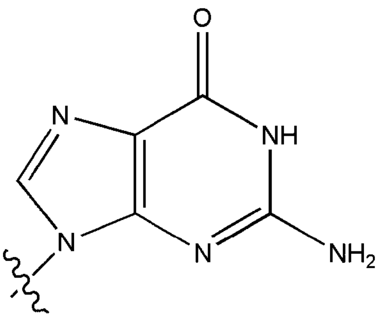, or 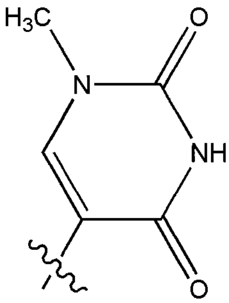.

In an embodiment, the $R_4$ is methoxy, and $R_5$ is OH.
In an embodiment, the compound of Chemical Formula 1 is a compound selected from the group consisting of compounds listed in Table 1 below:

[Table 1]

| Example | Structural Formula | Abbreviated Compound Name |
|---|---|---|
| 12 | 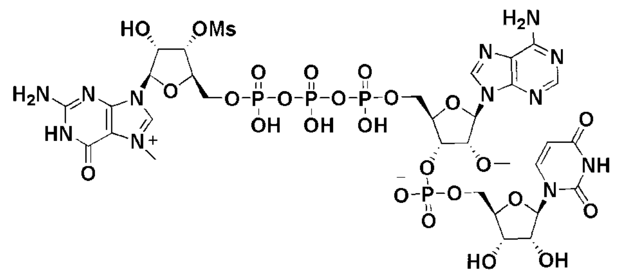 | $^{7m}G_{(3'OMs)}pppA_{(2'OMe)}pU$ |
| 16 | 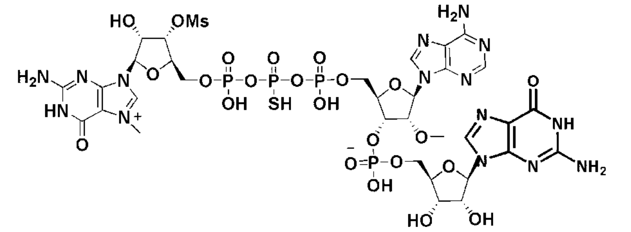 | $^{7m}G_{(3'OMs)}pp_{s}pA_{(2'OMe)}pG$ |
| 18 | 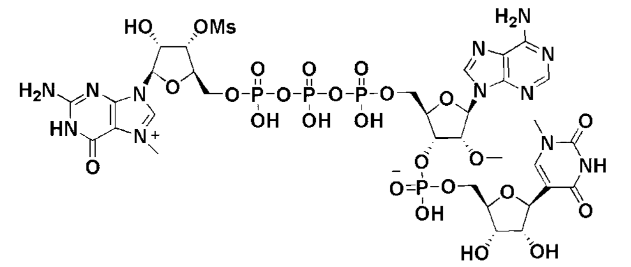 | $^{7m}G_{(3'OMs)}pppA_{(2'OMe)}p^{1m}\Psi$ |

In the abbreviated compound names, the G is guanosine, $^{7m}G$ is 7-methylguanosine, A is adenosine, U is uridine, $^{1m}\Psi$ is N1-methylpseudouridine, p is - P(=O)(OH)O-, $P_s$ is-P(=O)(SH)O-, Ms is mesyl, and Me is methyl.

The compound of Chemical Formula 1 may be prepared according to a method shown in examples. Those skilled in the art may prepare the compound of Chemical Formula 1 by appropriately modifying the method shown in the examples below, for example, by modifying conditions, order, and compounds of reaction.

The compound of Chemical Formula 1 according to an embodiment (compound 12) may be prepared by a method shown in Reaction Scheme 2a below.

[Reaction Scheme 2a]

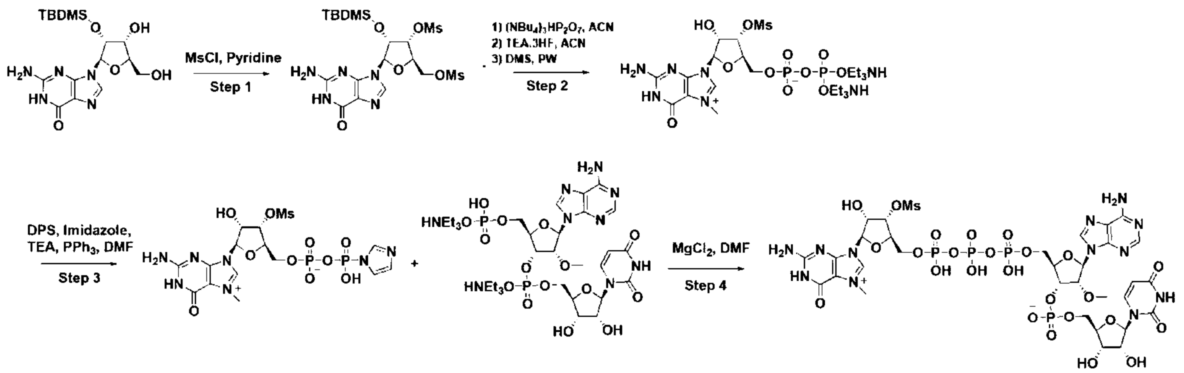


Details of the preparation method shown in the reaction scheme above are described in the following examples.

Reaction Scheme 2a may be expressed briefly with abbreviations below.

[Reaction Scheme 2a] $G_{(2'TBS)} \rightarrow G_{(2'TBS,\ 3'OMs,\ 5'OMs)} \rightarrow pp^{7m}G_{(3'OMs)} \rightarrow Im\text{-}pp^{7m}G_{(3'OMs)} + pA_{(2'OMe)}pU \rightarrow {}^{7m}G_{(3'OMs)}pppA_{(2'OMe)}pU$

Here, the G is guanosine, $^{7m}$G is 7-methylguanosine, A is adenosine, U is uridine, p is -P(=O)(OH)O-, TBS is tert-butyldimethylsilyl, Me is methyl, Im is imidazolide, and Ms is mesyl.

According to the preparation method, the compound of Chemical Formula 1 may be prepared by four steps. Thus, the compound of Chemical Formula 1 may be prepared more economically by reducing the number of steps compared to conventional methods for preparing cap analogs.

The compound of Chemical Formula 1 according to an embodiment may be used as a cap analog. Therefore, 5'-capped mRNA may be prepared by using the cap analog that is the compound of Chemical Formula 1.

Therefore, one particular aspect provides a cap analog that is the compound of Chemical Formula 1.

In addition, one particular aspect provides a method for preparing 5'-capped mRNA, the method including introducing a cap analog that is the compound of Chemical Formula 1 during synthesis of mRNA.

In addition, one particular aspect provides a composition or kit for preparing 5'-capped mRNA including the cap analog that is the compound of Chemical Formula 1.

In addition, one particular aspect provides a use of a cap analog that is the compound of Chemical Formula 1 for synthesis of mRNA.

In addition, one particular aspect provides mRNA 5'-capped with a cap analog that is the compound of Chemical Formula 1.

The method for preparing the 5'-capped mRNA by using the cap analog that is the compound of Chemical Formula 1 may be performed according to any method known in the art. In an embodiment, the 5'-capped mRNA may be prepared by the co-transcriptional capping method, which is a method of mRNA synthesis in which a chemically synthesized cap analog is simultaneously introduced during in vitro mRNA synthesis and the cap analog constitutes the 5' end. Specifically, the method may include: steps of introducing a cap analog that is a compound of Formula 1 into a mixture including an RNA polymerase under conditions in which transcription of a polynucleotide template occurs by the RNA polymerase; and incubating the mixture for a time sufficient to allow transcription of the template. A cap analog including a compound of Formula 1 according to an aspect may increase the efficiency of transcription of an in vitro mRNA compared to the efficacy of initiation utilizing standard GTP, ATP, CTP or UTP(or $^{1m}\Psi$TP), and subsequently increase the efficiency of protein expression of the transcribed capped mRNA during translation. Due to the increase in transcriptional efficiency, the synthesis of the mRNA may be increased by, for example, about 10 %, about 20 %, about 40 %, about 60 %, about 80 %, about 90 %, about 100 %, about 150 %, about 200 %, or about 500 % compared to synthesizing the mRNA by typical methods.

The cap analog according to an aspect, which is the compound of Chemical Formula 1, may significantly increase protein expression levels of 5'-capped mRNA molecules compared to the conventional TriLink's trinucleotide cap analog ($^{7m}G_{(3'OMe)}pppA_{(2'OMe)}pG$ or $^{7m}GpppA_{(2'OMe)}pU$). More specifically, mRNA molecules 5'-capped with a cap analog, in which 3'-OH residue of the compound of Chemical Formula 1 is monosulfonylated, may significantly increase initial protein expression after transfection compared to mRNA molecules 5'-capped by the conventional TriLink's trinucleotide 3'-methoxy cap analog ($^{7m}G_{(3'OMe)}pppA_{(2'OMe)}pG$ or $^{7m}GpppA_{(2'OMe)}pU$), thereby significantly increasing the overall protein expression levels (See Experimental Examples 2 to 6).

Particularly, since the cap analog of Example 16 ($^{7m}G_{(3'OMs)}pp_spA_{(2'OMe)}pG$) possesses a thio or thiol structure within the triphosphoryl structure, mRNA capped with said cap analog can more effectively inhibit the degradation of mRNA by decapping enzymes when administered in vivo, allowing the structure to remain stable for a long period of time. As a result, the cap analog of Example 16 can enable protein expression for a long duration in vivo and significantly increase the overall protein expression level.

The cap analog according to an aspect, which is the compound of Chemical Formula 1, has advantages of being synthesized economically in terms of time and cost by reducing the number of synthesis steps and column purifications compared to the TriLink's trinucleotide cap analog.

In more detail, by selecting an optimized starting material (e.g., $G_{(2'TBS)}$) in the preparation of the compound of Chemical Formula 1, the conventional two-step process of converting guanosine into guanosine monophosphate and then guanosine diphosphate may be simplified to a one-step process of directly converting guanosine into guanosine diphosphate, and the following process of 7-methylation of guanosine may be conducted in situ to shorten a crystallization/purification process. More specifically, by performing mesylation of 3' and 5'OH at the starting material ($G_{(2'TBS)}$) and then preparing 7-methylguanosine diphosphate ($pp^{7m}G$), the synthesis steps to 7-methylguanosine diphosphate ($pp^{7m}G$) may be shortened by two steps compared to the conventional TriLink's method for preparing the trinucleotide cap analog (method of [Reaction Scheme 1] below). In addition, while the TriLink's method for preparing the trinucleotide cap analog $^{7m}G_{(3'OMe)}pppN_{(2'OMe)}pN$ requires a total of 5 ion-exchange column purifications as shown in Reaction Scheme 1 below (to be purified: $pG_{(3'OMe)}$, $ppG_{(3'OMe)}$, $pp^{7m}G_{(3'Ome)}$, $pN_{(2'OMe)}pN$, and $^{7m}G_{(3'OMe)}pppN_{(2'OMe)}pN$) (See US 10,913,768), the compound of Chemical Formula 1 may be prepared by a total of 3 ion-exchange column purifications as shown in Reaction Scheme 2 below (to be purified: $pp^{7m}G_{(3'OR)}$, $pN_{(2'OMe)}pN$, and $^{7m}G_{(3'OR)}pppN_{(2'OMe)}pN$). Therefore, the cap analog of the compound of Chemical Formula 1 according to one aspect may be prepared by shortening the

synthesis process and using a significantly reduced number of purification processes compared to conventional cap analogs.

[Reaction Scheme 1] $G_{(3'OMe)} \rightarrow pG_{(3'OMe)} \rightarrow Im\text{-}pG_{(3'OMe)} \rightarrow ppG_{(3'OMe)} \rightarrow pp^{7m}G_{(3'OMe)} \rightarrow Im\text{-}pp^{7m}G_{(3'OMe)} + pN_{(2'OMe)}\ pN \rightarrow {}^{7m}G_{(3'OMe)}pppN_{(2'OMe)}pN$

[Reaction Scheme 2] $G_{(2'TBS)} \rightarrow G_{(2'TBS,\ 3'OR2,\ 5'OR3)} \rightarrow pp^{7m}G_{(3'OR)} \rightarrow Im\text{-}pp^{7m}G_{(3'OR)} + pN_{(2'OMe)}pN \rightarrow {}^{7m}G_{(3'OR)}pppN_{(2'OMe)}pN$

In Reaction Schemes 1 and 2, the G is guanosine, $^{7m}G$ is 7-methylguanosine, N is one of adenosine, cytidine, guanosine, uridine, and N1-methylseudouridine, p is - $P(=O)O_2$-, TBS is tert-butyldimethylsilyl, Me is methyl, Im is imidazolide, R2 and R3 are each independently a substituent selected from the group consisting of tosyl, brosyl, nosyl, esyl, mesyl, triflyl, tresyl, and dansyl groups.

Therefore, the cap analog of the compound of Chemical Formula 1 according to one aspect have advantages of further lowering manufacturing time and cost of the cap analog.

In an embodiment, according to the method for preparing the 5'-capped mRNA, at least one modified NTP may further be added to a transcription reaction. Modification of the at least one modified NTP does not substantially impair RNA polymerase-mediated synthesis of mRNA. The modified NTP may include, for example, at least one modified nucleoside base, at least one modified sugar, and at least one modified 5'-triphosphate. As such, the modified NTP may be incorporated onto the 3'-end of the cap analog, which supports additional elongation of a primer without blocking transcription. A modification group of the modified NTP may be a detectable label or detectable marker. Therefore, after transcription, prepared mRNA containing the detectable label or marker may be identified by size, mass, color, and/or affinity capture. In an embodiment, the detectable label or marker is a fluorescent dye, and the affinity capture label is biotin.

In an embodiment, one or more components of transcription reaction (cap analog and/or NTP) may be labeled with a detectable label or marker. Thus, after transcription, mRNA molecules may be identified, for example, by size, mass, affinity capture, and color. For example, the detectable label is a fluorescent dye, and the affinity capture label is biotin.

The kit or composition for preparing the 5'-capped mRNA may include all common transcription reagents for synthesis of mRNA (e.g., FLuc mRNA). More specifically, the kit may include: a cap analog; a container marked for transcription; instructions for performing mRNA synthesis; at least one reagent selected from the group consisting of at least one unmodified NTP, at least one modified NTP (e.g., methylpseudouridine 5'-triphosphate), RNA polymerase, other enzymes, reaction buffers, magnesium, and DNA templates.

The 5'-capped mRNA prepared by using the cap analog according to an embodiment includes the cap analog in the structure, and the 5'-capped mRNA may be used to express proteins in vivo by administering the 5'-capped mRNA into a living organism.

Therefore, another aspect provides a method for expressing a target peptide or protein in vivo, the method including administering mRNA including the cap analog into a living organism.

In addition, another aspect provides a pharmaceutical composition for expressing a target peptide or protein including mRNA containing the cap analog and a pharmaceutically acceptable carrier.

As used herein, the term "target peptide or protein" refers to any protein to be expressed in vivo. In the present disclosure, the mRNA capable of expressing the target peptide or protein is 5'-capped with the cap analog according to an aspect to express the target protein in vivo.

Depending on the types of the target peptide or protein, in vivo therapeutic or preventive effects on target diseases may be obtained. Therefore, the present disclosure may be used to treat or prevent any disease that may be treated or prevented by expression of the peptide or protein. Diseases that may be treated or prevented by expression of specific types of peptide or protein are known in the art, and the pharmaceutical composition can be used for the prevention or treatment of a target disease by inducing the expression of the peptide or protein.

In addition, another embodiment provides a medical use of the cap analog-containing mRNA for preventing or treating any disease on which in vivo expression of peptide or protein is effective.

The pharmaceutical composition and treatment or prevention method may be used for treatment or prevention by using gene replacement therapy, genome editing, cancer immunotherapy, or vaccination. In an embodiment, the pharmaceutical composition is a mRNA vaccine.

The pharmaceutical composition may be formulated for administration by injection or any suitable route known to those skilled in the art to treat or prevent a certain condition. An injectable composition includes, for example, sterile saline solution as a pharmaceutically acceptable carrier. The injectable composition may be formulated as a suspension in lipids or phospholipids, a ribosomal suspension, or an aqueous solution. Methods for formulating the pharmaceutical composition are well known to those skilled in the art.

In an embodiment, the pharmaceutical composition may include the cap analog-containing mRNA, as an active ingredient, in a concentration of about 0.01 % to 1 %. The concentration may vary according to frequency of administration,

dosage, administration method, and the like.

In an embodiment, the pharmaceutical composition may be administered to mammals, specifically, humans, the dosage may vary depending on individual's health condition, severity of disease, body weight, age, race, and the like, and professionals in the art may determine an appropriate dosage. In an embodiment, the dosage for humans may be in the range of 0.0001 to 100 mg/day, more specifically, the range of about 0.1 to 50 mg/day.

The cap analog-containing mRNA according to an embodiment may be introduced into cells in vivo or in vitro to express protein or peptide.

Therefore, another embodiment provides a cell including the cap analog-containing mRNA according to an aspect.

Another embodiment provides a cell including a protein or peptide translated from the cap analog-containing mRNA according to an aspect.

Methods for expressing proteins or peptides in cells in vivo or in vitro by using the mRNA are known in the art, and the proteins or peptides may be expressed in cells appropriately according to conventional methods.

[Examples]

Hereinafter, the present disclosure will be described in more detail with reference to the following examples. However, the following examples are merely presented to exemplify the present disclosure, and it will be apparent to those skilled in the art that modifications and variations could be made without departing from the scope and technical idea of the present disclosure as defined by the appended claims.

**Description of Abbreviation**

Abbreviations used herein are as follows.

TBDMS: tert-butyldimethylsilyl
Ms: methanesulfonyl
MsCl: methanesulfonyl chloride
TEA.3HF: triethylamine trihydrofluoride
THF: tetrahydrofuran
TBAP: tetra-n-butylammonium perchlorate
ACN: acetonitrile
DMS: dimethylsulfate
PW: purified water
DPS: 2,2'-dithiopyridine
TEA: triethylamine
$PPh_3$: triphenylphosphine
DMF: dimethylformamide
DMTr: 4,4'-dimethoxytrityl
DTT: dithiothreitol
IVT: in vitro transcription

Examples 1 to 3: **Preparation of 7-methyl, 3'-mesyloxy-guanosine 5'-diphosphate imidazolide** (Im-pp$^{7m}$G($_{3'OMesyloxy}$)) (3)

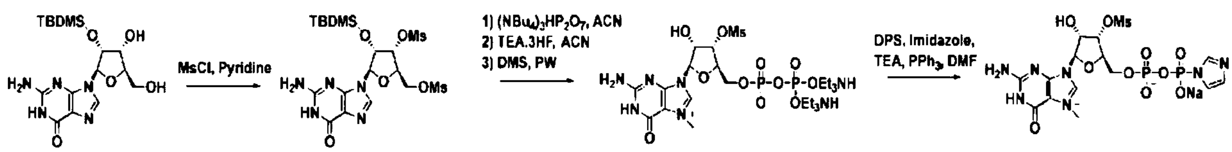


(1) (2) (3)

**Example** 1: **Preparation** of **2'-O-tert-butyldimethylsilyl, 3'mesyloxy, 5'mesyloxy guanosine** (1)

2'-O-tert-butyldimethylsilyl guanosine was commercially obtained and used as a starting material without further treatment. 2'-O-tert-butyldimethylsilyl guanosine (9.99 mmol) was added to 91 mL of anhydrous pyridine, followed by

stirring at 0 °C for 10 minutes while slowly adding methane sulfonyl chloride (29.97 mmol). Subsequently, the reaction solution was stirred at 10 to 20 °C for 3 hours. 270 mL of distilled water was slowly added thereto at 0 °C, followed by stirring at 5 to 10 °C for 1 hour. Crystals produced thereby were filtered, washed with distilled water, and then vacuum dried to obtain a target compound (1) (yield: 80 %).

$^1$H NMR (400 MHz, DMSO-d6, 25 °C): d= 10.72 (s, 0.84H), 7.96 (s, 1H), 6.56 (1.65H), 5.80 (d, 1H), 5.15 (d, 1H), 4.96 (m, 1H), 4.56 ~ 4.62 (m, 3H, overlapped), 3.32 (m, 3H), 3.25 (m, 3H), 0.699 (m, 9H), -0.014 (m, 3H), -0.231 (m, 3H).

**Example 2: Preparation of 7-methyl-3'-mesyloxy guanosine 5'-triethylammonium diphosphate (2)**

2'-O-tert-butyldimethylsilyl, 3'mesyloxy, 5'mesyloxy guanosine (1) (7.59 mmol) was dissolved in 33.6 mL of anhydrous acetonitrile, and tris(tetrabutylammonium) hydrogen pyrophosphate (19.73 mmol) was added thereto, followed by stirring at room temperature for 48 hours. Then, the reaction solution was stirred for 1 hour at room temperature while slowing adding triethylamine trihydrofluoride (22.77mmol) thereto. After washing by adding 42 mL of dichloromethane and 42 mL of purified water to the reaction solution, an aqueous layer was separated therefrom. After washing the separated aqueous layer by adding 42 mL of dichloromethane thereto, the aqueous layer was separated again. The pH of the separated aqueous layer was adjusted to 4.0 by using glacial acetic acid. Dimethylsulfate (303.6 mmol) was slowly added to the reaction solution for 30 minutes, followed by stirring at room temperature for 3 hours. In this regard, the pH of the reaction solution was maintained at 4.0±0.5 by using a 1 M sodium hydroxide. The reaction solution was subject to extraction three times by using 126 mL of dichloromethane to remove unreacted dimethylsulfate, and then the aqueous layer was separated. The separated aqueous layer was titrated to pH 5.5 by using 1 M sodium hydroxide and the target compound was separated by using a DEAE Sepharose column (140 x 210 mm) and freeze-dried to obtain a triethylammonium salt of the target compound (2) (yield 30.0 %).

$^1$H NMR (400 MHz, $D_2O$, 25 °C): d= 6.11 (d, 1H), 5.44 (dd, 1H), 5.02 (m, 1H), 4.74 (m, $D_2O$ overlapped, 1H), 4.23 (m, 2H), 4.1 (s, 3H), 3.32 (s, 3H), 3.19 ~ 3.13 (m, 12H), 1.28 ~ 1.18 (m, 18H) $^{31}$P NMR (162 MHz, $D_2O$, 25 °C): d= -7.13 (d, 1P), -10.77 (d, 1P).

**Example 3: Preparation of 7-methyl-3'-mesyloxy guanosine 5'-diphosphate imidazolide (3)**

7-methyl-3'-mesyloxy guanosine 5'-triethylammonium diphosphate (2) (1.36 mmol) was dissolved in 40 mL of dimethylformamide, and imidazole (13.6 mmol) and 2,2'-dipyridyl disulfide (6.8 mmol) were added thereto. Triethylamine (1.36 mmol) and triphenylphosphine (6.8 mmol) were added to the reaction solution, followed by stirring at room temperature for 2 hours. The reaction solution was added to a solution of sodium perchlorate (2.72 mmol) dissolved in 320 mL of acetone, cooled to 4 °C, and then crystals produced thereby were filtered, washed with cold acetone, and vacuum dried to obtain a sodium salt of the target compound (3) (yield 100.0 %).

$^1$H NMR (400 MHz, $D_2O$, 25 °C): d= 7.999 (s, 1H), 7.32 (s, 1H), 7.07 (s, 1H), 6.06 (d, 1H), 5.29 (m, 1H), 4.90 (m, 1H), 4.67 (m, 1H), 4.21 ~ 4.08 (m, 2H), 4.05 (s, 3H), 3.29 (s, 3H) $^{31}$P NMR (162 MHz, $D_2O$, 25 °C): d= -11.41 (d, 1P), -19.61 (d, 1P).

**Examples 4 to 7: Preparation of $pA_{(2'OMe)}pG$ (7)**

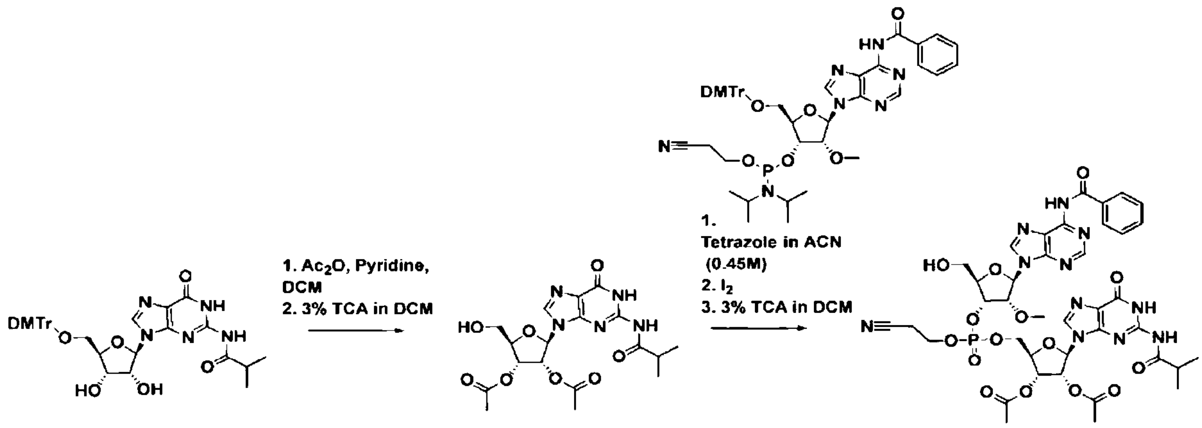


(4) (5)

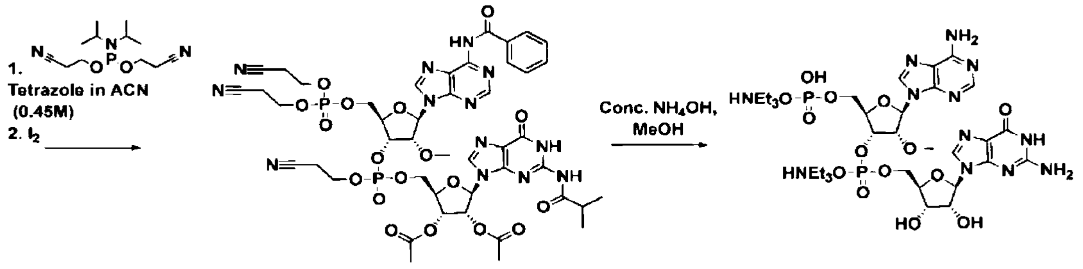


(6) (7)

**Example 4: Preparation of N2-isobutyryl-2',3'-diacetoxy-guanosine (4)**

After N2-isobutyryl-5'-O-DMT guanosine (3.66 mmol) was dissolved in 12 mL of dichloromethane, pyridine (18.3 mmol) and acetic anhydride (18.3 mmol) were added thereto, followed by stirring at room temperature for 5 hours. The reaction solution was subject to extraction by adding 24 mL of ethyl acetate thereto, followed by washing with 14.4 mL of a saturated sodium bicarbonate aqueous solution, 14.4 mL of a 20 % citric acid aqueous solution, and 14.4 mL of distilled water. An organic layer was dried with sodium sulfate and distilled under reduced pressure, and the residue was dried under nitrogen for one day. 36 mL of a 3 % trichloroacetic acid aqueous solution was added to the dried product and reacted at room temperature for 3 hours, and then 24 mL of methanol was added thereto and further reacted for 2 hours and 30 minutes. After the reaction solution was distilled under reduced pressure, 36 mL of dichloromethane and 18 mL of distilled water were added thereto, and then a saturated sodium bicarbonate aqueous solution was added thereto for neutralization. An organic layer was separated, dried with sodium sulfate, and distilled under reduced pressure. The residue distilled under reduced pressure was dissolved in 12 mL of ethylacetate and crystallized by slowly adding the solution to 108 mL of hexene. Crystals produced thereby were filtered, repeatedly washed 3 times with 12 mL of hexene, and vacuum dried to obtain a target compound (4) (yield 89.6 %). LC-MS (ESI, m/z) = 438.16 [M + $H^+$]

**Example 5: Preparation of (N2-isobutyryl-2',3'-diacetoxy-guanosinyl)-N6-benzoyl-2'-methoxy-adenosinyl cyanoethyl phosphate ester (5)**

5'-O-DMT-N6-benzoyl-2'-methoxy-adenosine amidite was commercially obtained and used as a starting material without further treatment. N2-isobutyryl-2',3'-diacetoxy-guanosine (4) (3.28 mmol) and 5'-O-DMT-N6-benzoyl-2'-methoxy-adenosine amidite (4.26 mmol) were dissolved in 1H-tetrazole (0.45 M acetonitrile solution, 12.79 mmol) and stirred at room temperature for 1 hour. A solution, prepared by dissolving iodine (2.15 mmol) in 56.7 mL of a tetrahydrofuran:distilled water:pyridine mixed solution (v:v:v, 7:2:1), was added to the reaction solution and stirred for 45 minutes. After adding 7.2 mL of a 10 % sodium thiosulfate aqueous solution to the reaction solution, 43 mL of dichloromethane and 14.3 mL of distilled water were added thereto, and an organic layer was separated therefrom. The separated organic layer was dried with sodium sulfate, distilled under reduced pressure, and dried under nitrogen for one day. 21.5 mL of a 3 % trichloroacetic acid aqueous solution was added to the dried product and reacted at room temperature for 3 hours, and then 14.3 mL of methanol was added thereto and further reacted for 2 hours and 30 minutes. After the reaction solution was distilled under reduced pressure, 21.5 mL of dichloromethane and 10.7 mL of distilled water were added thereto, and then a saturated sodium bicarbonate aqueous solution was added thereto for neutralization. An

organic layer separated therefrom was washed with distilled water, dried with sodium sulfate, and distilled under reduced pressure. The residue distilled under reduced pressure was dissolved in 28.6 mL of dichloromethane and crystallized by slowing adding 86 mL of methyl tert-butyl ether at room temperature. Crystals produced thereby were filtered and vacuum dried for one day to obtain a target compound (5) (yield 87.8 %). LC-MS (ESI, m/z) = 938.28 [M + $H^+$]

**Example 6: Preparation of p(OCE)$_2$A$^{bz}$(2'OMe)p(OCE)G$^{ib}$(2',3'OAc) (6)**

(N2-isobutyryl-2',3'-diacetoxy-guanosinyl)-N6-benzoyl-2'-methoxy-adenosinyl cyanoethyl phosphate ester (5) (2.13 mmol), bis(2-cyanoethyl)-N,N-diisopropylphosphoamidite (4.26 mmol) were dissolved in 9.47 mL of 1H-tetrazole (0.45 M acetonitrile solution, 4.26 mmol) and stirred at room temperature for 30 minutes. A solution obtained by dissolving iodine (3.2 mmol) in 30 mL of a tetrahydrofuran:distilled water:pyridine mixed solution (v:v:v, 7:2:1) was added to the reaction solution and stirred for 30 minutes. After adding 20 mL of a 10 % sodium thiosulfate aqueous solution to the reaction solution, 100 mL of dichloromethane and 40 mL of distilled water were added thereto, and then an organic layer was separated therefrom. The separated organic layer was dried with sodium sulfate and distilled under reduced pressure. A concentrate obtained by the distillation under reduced pressure was dissolved in 27 mL of dichloromethane and crystallized by slowly adding the solution to 133 mL of methyl tert-butyl ether at room temperature. Crystals produced thereby were filtered and vacuum dried for one day to obtain a target compound (6) (yield 92.2 %). LC-MS (ESI, m/z) = 1124.30 [M + $H^+$]

**Example 7: Preparation of pA$_{(2'OMe)}$pG (7)**

p(OCE)$_2$A$^{bz}$(2'OMe)p(OCE)G$^{ib}$(2',3'OAc) (6) (1.78 mmol) was added to a mixed solution of 44 mL of methanol and 44 mL of conc. ammonia and stirred at 50 to 55 °C for 24 hours. Upon completion of the reaction, the solvent was distilled under reduced pressure, and 20 mL of methanol was added to the concentrate, followed by distillation 3 times. The concentrate was dissolved in distilled water and purified by using a DEAE Sepharose column and reversed phase chromatography to obtain a triethylammonium salt of the target compound (7) (yield 70 %).

$^1$H NMR (400 MHz, $D_2O$, 25 °C): d= 8.44 (s, 1H), 8.12 (s, 1H), 7.90 (s, 1H), 6.07 (m, 1H), 5.80 (m, 1H), 4.46 ~ 4.01 (m, 8H), 3.45 (m, 3H) LC-MS (ESI, m/z) = 707.13 [M + H+]

**Examples 8 to 11: Preparation of pA$_{(2'OMe)}$pU (8)**

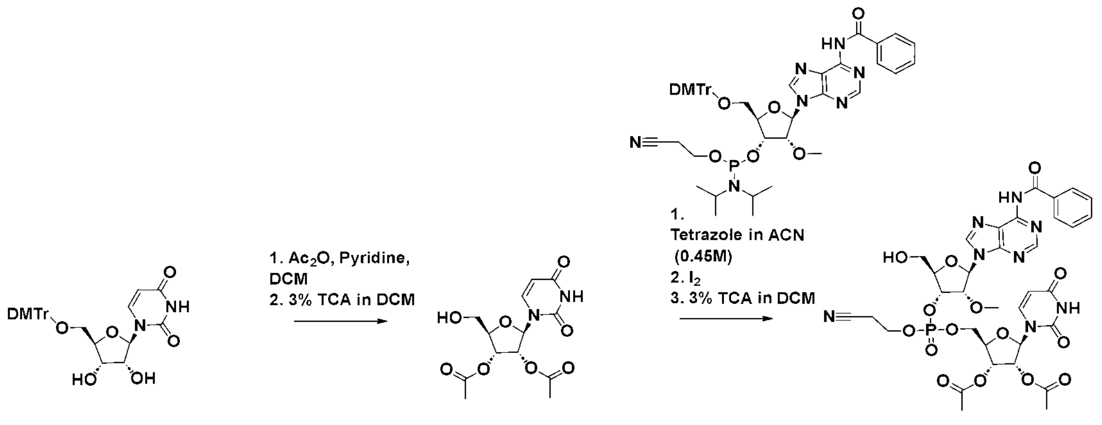


(8) (9)

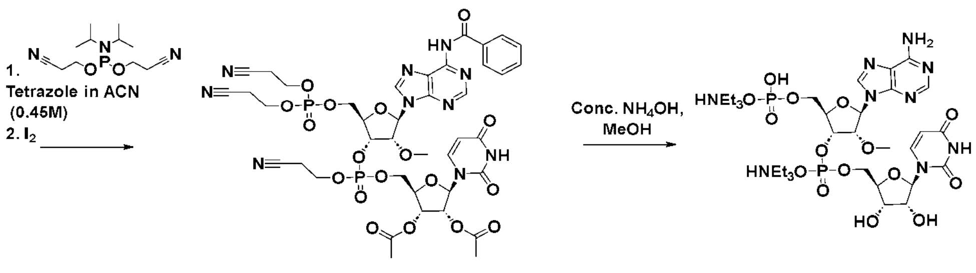


(10) (11)

**Example 8: Preparation of 2,3'-diacetoxy-uridine (8)**

5'-O-DMT-uridine (4.39 mmol) was dissolved in 12 mL of dichloromethane, and then pyridine (21.95 mmol) and acetic anhydride (21.95 mmol) were added thereto, followed by stirring at room temperature for 5 hours. The reaction solution was extracted by adding 24 mL of ethyl acetate and then washed with 14.4 mL of a saturated aqueous sodium bicarbonate solution, 14.4 mL of a 20% aqueous citric acid solution, and 14.4 mL of distilled water. The organic layer was dried over sodium sulfate and distilled under reduced pressure, and the residue was dried under nitrogen for one day. 36 mL of a 3% aqueous trichloroacetic acid solution was added to the dried product and reacted at room temperature for 3 hours, and then 24 mL of methanol was added thereto, followed by additional stirring for 2 hours and 30 minutes. The reaction solution was distilled under reduced pressure, 36 mL of dichloromethane and 18 mL of distilled water were added, and then a saturated aqueous sodium bicarbonate solution was added to neutralize the mixture. The organic layer was separated, dried over sodium sulfate, and distilled under reduced pressure. The residue distilled under reduced pressure was dissolved in 12 mL of ethyl acetate and crystallized by slowly adding the solution to 108 mL of hexene at room temperature. The resulting crystals were filtered, repeatedly washed three times with 12 mL of hexene, and vacuum-dried to obtain the target compound (8) (yield 79.4%). LC-MS (ESI, m/z) = 328.28 [M + $H^+$]

**Example 9: Preparation of (2,3'-diacetoxy-uridyl)-N6-benzoyl-2'-methoxy-adenosinyl cyanoethyl phosphate ester (9)**

5'-O-DMT-N6-benzoyl-2'-methoxy-adenosine amidite was commercially obtained and used as a starting material without further treatment. 2,3'-diacetoxy-uridine (8) (4.36 mmol) and 5'-O-DMT-N6-benzoyl-2'-methoxy-adenosine amidite (5.67 mmol) were dissolved in 28.4 mL of 1H-tetrazole (0.45 M acetonitrile solution, 12.79 mmol) and stirred at room temperature for 1 hour. After iodine (2.83 mmol) was dissolved in 56.7 mL of a mixed solution of tetrahydrofuran:distilled water:pyridine (v:v:v, 7:2:1), the solution was added to the reaction solution and stirred for 45 minutes. 7.2 mL of a 10% aqueous sodium thiosulfate solution was added to the reaction solution, followed by the addition of 43 mL of dichloromethane and 14.3 mL of distilled water, and the organic layer was separated. The separated organic layer was dried over sodium sulfate, distilled under reduced pressure, and then dried under nitrogen for one day. 21.5 mL of a 3% aqueous trichloroacetic acid solution was added to the dried product and reacted at room temperature for 3 hours, and then 14.3 mL of methanol was added thereto, followed by additional stirring for 2 hours and 30 minutes. The reaction solution was distilled under reduced pressure, 21.5 mL of dichloromethane and 10.7 mL of distilled water were added, and then a saturated aqueous sodium bicarbonate solution was added to neutralize the mixture. The separated organic layer was

washed with distilled water, dried over sodium sulfate, and distilled under reduced pressure. The residue distilled under reduced pressure was dissolved in 28.6 mL of dichloromethane and crystallized by slowly adding the solution to 86 mL of methyl tert-butyl ether at room temperature. The resulting crystals were filtered and vacuum-dried for one day to obtain the target compound (9) (yield 80%). LC-MS (ESI, m/z) = 828.68 [M + H+]

**Example 10: Preparation of $p(OCE)_2A^{bz}$(2'OMe)p(OCE)U(2,3'OAc) (10)**

(2',3'-diacetoxy-uridyl)-N6-benzoyl-2'-methoxy-adenosinyl cyanoethyl phosphate ester (9) (2.41 mmol) and bis(2-cyanoethyl)-N,N-diisopropylphosphoramidite (4.82 mmol) were dissolved in 10.7 mL of 1H-tetrazole (0.45 M acetonitrile solution, 4.26 mmol) and stirred at room temperature for 30 minutes. After iodine (3.6 mmol) was dissolved in 30 mL of a mixed solution of tetrahydrofuran:distilled water:pyridine (v:v:v, 7:2:1), the solution was added to the reaction solution and stirred for 30 minutes. 20 mL of a 10% aqueous sodium thiosulfate solution was added to the reaction solution, followed by the addition of 100 mL of dichloromethane and 40 mL of distilled water, and the organic layer was separated. The separated organic layer was dried over sodium sulfate and distilled under reduced pressure. The concentrated residue obtained from the distillation was dissolved in 27 mL of dichloromethane and crystallized by slowly adding the solution to 133 mL of methyl tert-butyl ether at room temperature. The resulting crystals were filtered and vacuum-dried for one day to obtain the target compound (10) (yield 90%). LC-MS (ESI, m/z) = 1014.79 [M + H+]

**Example 11: Preparation of $pA_{(2'OMe)}pU$ (11)**

$p(OCE)_2A^{bz}$(2'OMe)p(OCE)U(2,3'OAc) (10) (1.97 mmol) was added to a mixed solution of 44 mL of methanol and 44 mL of concentrated ammonia, followed by stirring at 50 to 55°C for 24 hours. Upon completion of the reaction, the solvent was distilled under reduced pressure, and 20 mL of methanol was added to the concentrate, followed by distillation three times. The concentrate was redissolved in distilled water and purified using a DEAE Sepharose column and reverse-phase chromatography to obtain the triethylammonium salt of the target compound (11) (yield 70%). LC-MS (ESI, m/z) = 667.42 [M + H+]

**Example 12: Preparation of $^{7m}G_{(3'OMs)}pppA_{(2'OMe)}pU$ (12)**

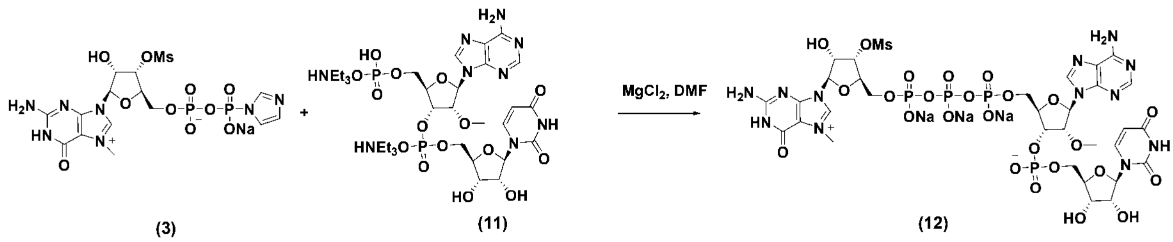


(3) (11) (12)

Magnesium chloride was added to dimethylformamide and dissolved. Im-$pp^{7m}G_{(3'OMs)}$ (3) (0.99 mmol) and $pA_{(2'OMe)}pU$ (11) (0.55 mmol) were added to the reaction solution, followed by stirring at room temperature for 24 hours. Upon completion of the reaction, 275 mL of a 25 mM aqueous ethylenediaminetetraacetic acid (EDTA) solution was added dropwise to terminate the reaction. The mixture was cooled to room temperature and neutralized with a 1M aqueous sodium bicarbonate solution. The reaction solution was purified using a DEAE Sepharose column (140 x 210 mm), distilled, and vacuum-dried. The dried solid was dissolved in 2.5 mL of distilled water and added to a solution of sodium perchlorate (2.44 mmol) in 15 mL of acetone. After cooling to 4°C, the resulting crystals were filtered, washed with cold acetone, and vacuum-dried to obtain the sodium salt of the target compound (12) (yield 60.0%).

$^1$H NMR (400 MHz, $D_2O$, 25°C): δ= 8.33 (s, 1H), 8.09 (s, 1H), 7.81 (s, 1H), 5.97 (s, 1H), 5.87 (d, 1H), 5.79 (d, 1H), 5.70(d, 1H), 5.30 (m, 1H), 4.79 ~ 4.12 (m, overlapped, 15H), 4.00 (s, 3H), 3.44 (s, 3H), 3.28 (s, 3H) $^{31}$P NMR (162 MHz, $D_2O$, 25°C): δ= -0.48 (s, 1P), -11.03 (dd, 2P), -22.36 (t, 1P). LC-MS (ESI, m/z) = 1184.72 [M + H+].

**Examples 13 to 14: Preparation of 7-methyl-3'-mesyloxy guanosine 5'-phosphorothioate triethylammonium salt (14)**

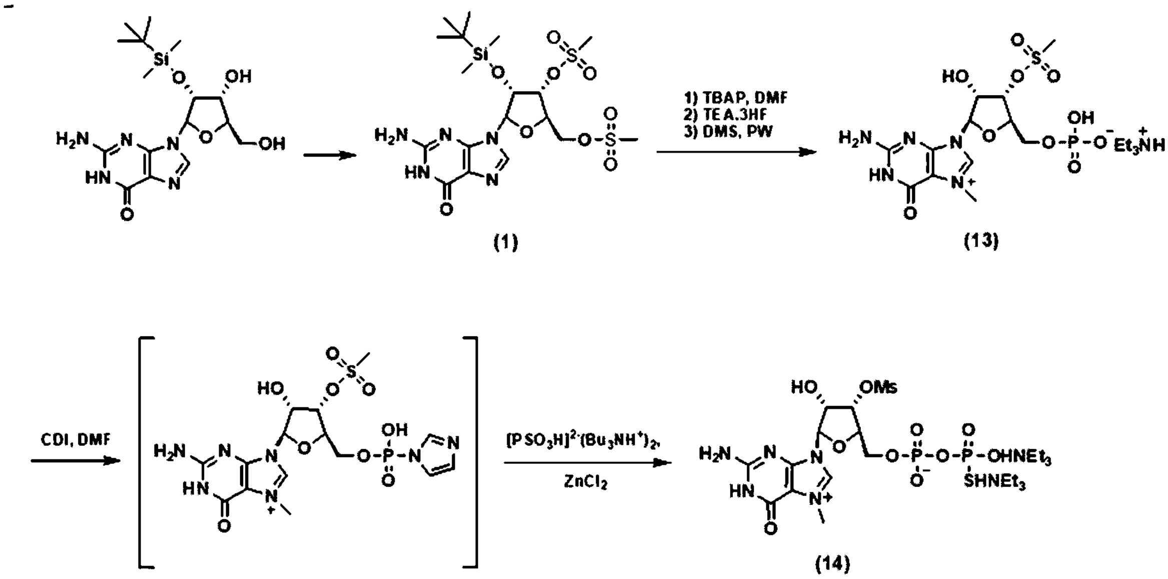


(1) (13) (14)

Example 13: Preparation of 7-methyl-3'-mesyloxy guanosine 5'-phosphate triethylammonium salt (13)

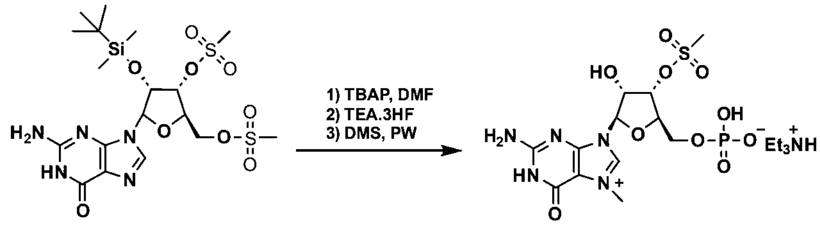


(1)
(G (2'-TBS, 3',5'-OMs))

(13)
(m7Gp)

TBAP (18.1 mmol) was added to 15 mL of dimethylformamide, and the temperature was raised to 50°C. 2'-O-tert-butyldimethylsilyl, 3'-mesyloxy, 5'-mesyloxy guanosine (1) (1.8 mmol) was added, and the mixture was stirred at 50°C for 1 hour. After the reaction was complete, the mixture was cooled to 20-25°C, and triethylamine trihydrofluoride (TEA-HF) (9.0 mmol) was added, followed by stirring at 20-25°C for at least 12 hours. Upon completion of the reaction, 150 mL of purified water were added to the reaction solution, and then 150 mL of dichloromethane were added to the reaction solution, stirred, and allowed to stand to separate the aqueous layer. The separated aqueous layer was concentrated under reduced pressure to remove the solvent, and after distillation, the remaining residue was dissolved by adding 20 mL of purified water, followed by the addition of dimethyl sulfate (54.2 mmol) and stirring at 20-25°C for 2-3 hours. After completion of the reaction, the pH was adjusted to 5.5 using 1M NaOH aqueous solution, and the target compound was separated and purified using an anion column and lyophilized to obtain the target compound, 7-methyl-3'-mesyloxy guanosine 5'-phosphate triethylammonium salt (13) (yield 30.1%).

**Example 14: Preparation of 7-methyl-3'-mesyloxy guanosine 5'-phosphorothioate triethylammonium salt (14)**

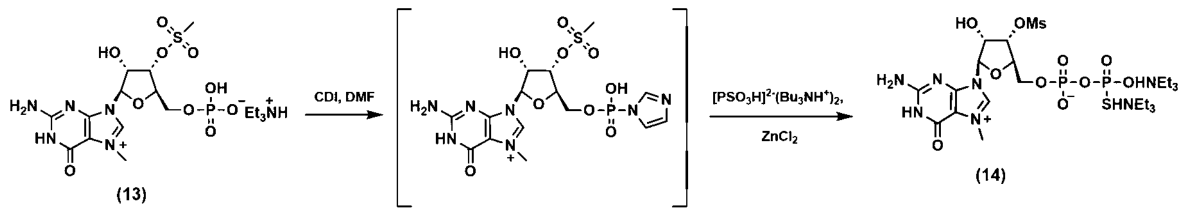


(13) (14)

7-methyl-3'-mesyloxy guanosine 5'-phosphate triethylammonium salt (13) (5.4 mmol) and CDI (32.3 mmol) were added to 45 mL of dimethylformamide in Reactor 1, followed by stirring at 20-25°C for 3-5 hours. Upon completion of the reaction, dry methanol (26.9 mmol) was added to the reaction solution and stirred for 5 minutes, after which $[PSO_3H]_2$-$(Bu_3NH^+)_2$ (32.3 mmol) diluted in 60 mL of dimethylformamide was added. Zinc chloride (43.0 mmol) was then added and the mixture was stirred at 20-25°C for 25 minutes. In Reactor 2, a solution of EDTA (43.0 mmol) diluted in 450 mL of purified water was cooled to 5-10°C, and the solution from Reactor 1 was added to the solution in Reactor 2. After confirming that the pH was 6.0 or higher by adding 2M TEAB solution, the pH was adjusted to 8.0 by adding additional 2M TEAB solution. The target compound was separated and purified using an anion column and lyophilized to obtain the target compound, 7-methyl-3'-mesyloxy guanosine 5'-phosphorothioate triethylammonium salt (14) (yield 43.6%).

**Example 15: Preparation of Im-pA$_{(2'Ome)}$pG (15)**

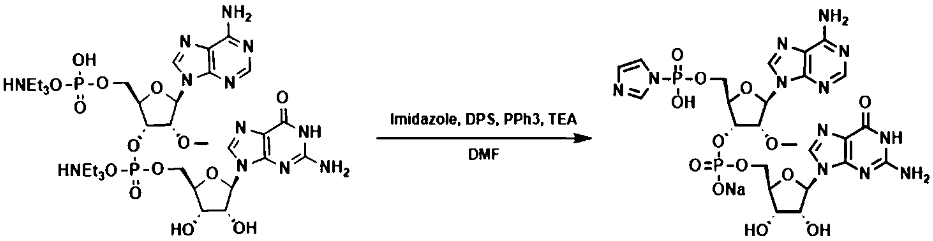


(7) (15)

pA$_{(2'OMe)}$pG (7) (2.20 mmol) prepared in Example 7, imidazole (35.2 mmol), DPS (2,2'-dithiodipyridine) (13.2 mmol), and triphenylphosphine (13.2 mmol) were added to 44 mL of dimethylformamide in Reactor 1 and stirred. Triethylamine (6.6 mmol) was added, followed by stirring at 20-25°C for 3-20 hours. After the reaction was complete, a sodium perchlorate solution, prepared by adding sodium perchlorate (22.0 mmol) to 440 mL of acetonitrile, was added to the reaction solution in Reactor 1 to precipitate crystals. The crystals were separated by filtration to remove the solution and vacuum-dried to obtain the target compound, Im-pA$_{(2' OMe)}$pG (15) (yield 89.5%).

**Example 16: Preparation of $^{7m}G_{(3' OMs)}pp_spA_{(2' OMe)}pG$ (16)**

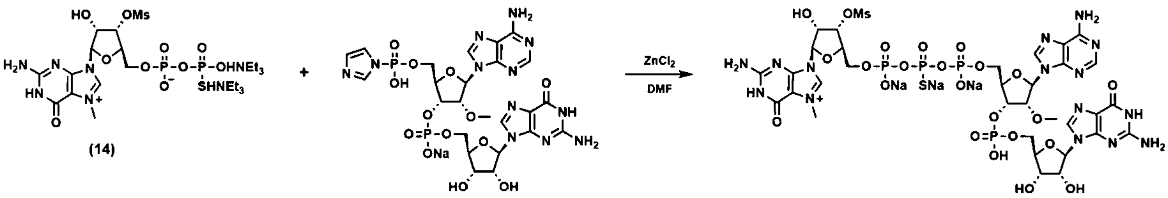


(14) + (15) (16)

Zinc chloride ($ZnCl_2$) (6.501 mmol), Im-pA$_{(2'OMe)}$pG (15) (0.4063 mmol), and 7-methyl-3'-mesyloxy guanosine 5'-phosphorothioate triethylammonium salt (14) (0.6582 mmol) were added to 6.64 mL of dimethylformamide in Reactor 1, followed by stirring at 20-25°C for 12-50 hours. Upon completion of the reaction, an EDTA solution prepared by adding EDTA (6.501 mmol) to 47.4 mL of purified water was added to the reaction solution in Reactor 1. After stirring, the pH was adjusted to 6.0-7.0 by adding a saturated aqueous sodium bicarbonate solution. The target compound was separated and purified using an anion column and lyophilized. The dried solid was dissolved in 2.5 mL of distilled water and added to a solution of sodium perchlorate (3.95 mmol) in 15 mL of acetone. After cooling to 4°C, the resulting crystals were filtered, washed with cold acetone, and vacuum-dried to obtain the sodium salt of the target compound (16) (yield 61.7%).

1H NMR (400 MHz, D2O, 25°C): δ= 8.28 (s, 1H), 8.05 (s, 1H), 7.89 (s, 1H), 5.92 (d, 1H), 5.82 (d, 1H), 5.77 (d, 1H), 5.33 (m, 1H), 4.59 ~ 4.12 (m, overlapped, 13H), 4.00 (s, 3H), 3.39 (s, 3H), 3.29 (s, 3H), 1.15 (d, 1H) 31P NMR (162 MHz, D2O, 25°C): δ= 30.43 (t, 1P), -0.37 (s, 1P), -11.84 (dd, 2P). LC-MS (ESI, m/z) = 1240.83 [M + H+].

**Example 17: Preparation of $pA_{(2'OMe)}p^{1m\Psi}$ (17)**

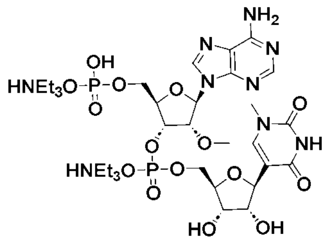

The compound $pA_{(2'OMe)}p^{1m\Psi}$ was prepared in the same manner as the preparation method of $pA_{(2'OMe)}pU$ in Examples 8 to 11, except that 5'-O-DMT-N1-methylpseudouridine was used as the starting material.

**Example 18: Preparation of $^{7m}G_{(3'OMs)}pppA_{(2'OMe)}p^{1m\Psi}$ (18)**

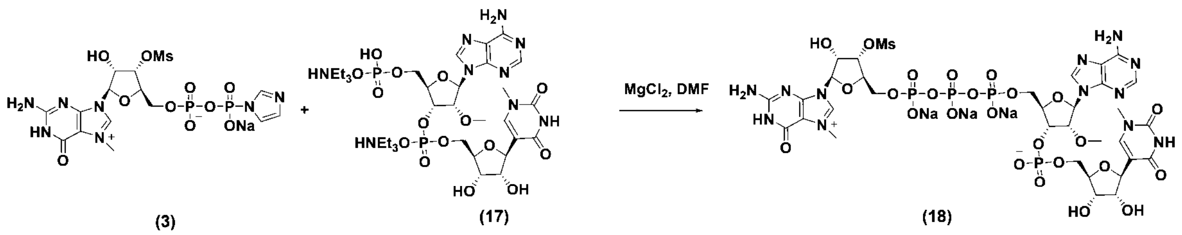


(3) (17) (18)

The compound (18) was prepared in the same manner as Example 12, except that $pA_{(2'OMe)}p^{1m\Psi}$ of Example 17 was used as a starting material instead of $pA_{(2'OMe)}pU$ (11) used as the starting material in Example 12, and the product was concentrated using a C18 column (50 x 250 mm) after purification using a DEAE Sepharose column (140 x 210 mm). Thus, the title reaction product (18) (yield 70%) was prepared.

$^{1}H$ NMR (400 MHz, D2O, 25°C): δ= 8.52 (s, 1H), 8.18 (s, 1H), 7.95(s, 1H), 6.16 (d, 1H), 5.96 (d, 1H), 5.05 (m, 1H), 4.89 (m, 1H), 4.70 ~ 4.02 (m, overlapped, 14H), 4.00 ~ 3.41 (m, 9H), 3.16 (s, 3H) 31P NMR (162 MHz, D2O, 25°C): δ= -0.48 (s, 1.65P), -10.82 ~ -11.04 (dd, 3.3P), -22.15 ~ -22.39 (t, 1.65P). LC-MS (ESI, m/z) = 1195.71 [M + H+].

**Experimental Example 1: In Vitro Transcription of Capped Luciferase mRNA for Co-transcriptional Capping**

For mRNA synthesis reactions using compounds 12, 16, or 18 as cap analogs, a transcription reaction mixture was prepared comprising: 100 ng/mL firefly luciferase DNA transcription template (including poly A 101), 5 mM ATP, CTP, GTP, and UTP (or $^{1m\Psi}$TP (N1-Methyl-Pseudouridine 5'-Triphosphate)), 4 mM of compound 12, 16, or 18, 10 U/mL T7 RNA polymerase (New England Biolab, #dy1670), 1 U/mL RNase inhibitor protein, 0.01 U/mL inorganic pyrophosphatase, 40 mM Tris·HCl (pH 8.0), 20 mM magnesium acetate, 1 mM spermidine, and 10 mM DTT. As comparative examples, transcription reaction mixtures were prepared under the same conditions by adding $^{7m}G_{(3'OMe)}pppA_{(2'OMe)}pG$, TriLink's trinucleotide cap analogs or $^{7m}GpppA_{(2'OMe)}pU$. Each prepared transcription reaction mixture was reacted at 37°C for 1-4 hours. Subsequently, to terminate the reaction, 10 mM Tris·HCl (pH 7.5), 2.5 mM magnesium chloride, 0.1 mM calcium chloride, and 100 U/mL DNase I (Thermo Fisher, #2270A) were added to the reactants, followed by a reaction at 25°C for 1 hour. The resulting mRNA was purified using the Monarch® RNA Cleanup Kit from New England Biolabs according to the manufacturer's instructions, or reverse-phase high-performance liquid chromatography (RP-HPLC). The concentration of the purified mRNA was measured using a Thermo Scientific Nanodrop spectrophotometer. Thereafter, the capping efficiency for each cap analog was calculated according to [Equation 1] using LC-MS, and the results are shown in Table 1.

[Equation 1]

$$\text{Capping Efficiency (\%)} = \frac{\text{Sum of Peak Areas of Capped mRNA}}{\text{Sum of Peak Areas of Capped mRNA} + \text{Sum of Peak Areas of Uncapped mRNA}} \times 100$$

According to the results in Table 1, it was confirmed that the IVT (in vitro transcription) yields of compounds 12, 16,

and 18 were nearly equivalent to the IVT yields of $^{7m}G_{(3'OMe)}pppA_{(2'OMe)}pG$, conventional third-generation cap analogs from TriLink, and $^{7m}GpppA_{(2'OMe)}pU$. Therefore, it was confirmed that the compounds according to one embodiment can be used to synthesize 5'-capped mRNA with a yield equivalent to that of $^{7m}G_{(3'OMe)}pppA_{(2'OMe)}pG$ or $^{7m}GpppA_{(2'OMe)}pU$, conventional third-generation cap analogs.

[Table 2]

| Cap analog | Capping Efficiency (%) |
|---|---|
| $^{7m}G_{(3'OMe)}pppA_{(2'OMe)}pG$ | 99.24 |
| $^{7m}GpppA_{(2'OMe)}pU$ | 100.00 |
| Compound 12 | 95.00 |
| Compound 16 | 98.95 |
| Compound 18 | 100.00 |

**Experimental Example 2: In Vitro Expression Test of Luciferase mRNA Capped with Compound 12**

After encapsulating the luciferase mRNA capped with Compound 12, which was prepared in Experimental Example 1, into LNPs, the luciferase expression of the mRNA-LNP complex was confirmed in HEK293S cells.

To prepare the mRNA-LNP complex, an aqueous luciferase mRNA solution dissolved in 100 mM sodium acetate (pH 4.0) was mixed with a lipid mixture containing an ethanol phase of SM-102 (Cayman), DSPC (Avanti), Cholesterol (Sigma), and DMG-PEG2000 (Cayman). Using the PreciGenome Flex M model, luciferase mRNA-LNP complexes capped with Compound 12 or $^{7m}GpppA_{(2'OMe)}pU$ were prepared. Subsequently, the prepared luciferase mRNA-LNP complexes were purified using an Amicon® Ultra-15 centrifugal filter.

The luciferase mRNA-LNP complexes capped with Compound 12 or $^{7m}GpppA_{(2'OMe)}pU$ prepared in the previous step were transfected into HEK293S cells. Specifically, HEK293S cells were seeded in a 12-well plate at a density of $1.0 \times 10^6$ cells per well, treated with 3 $\mu g$ of the luciferase mRNA-LNP complexes capped with Compound 12 or $^{7m}GpppA_{(2'OMe)}pU$, mixed using a pipette, and then incubated overnight. To confirm luciferase expression, $0.05 \times 10^6$ cells were harvested from each well at 24, 48, and 72 hours post-transfection. Luciferase expression was measured using the Steady-Glo Luciferase Assay System (Promega, #E2520) according to the manufacturer's manual. After confirming the total luciferase expression levels, significance was verified using a t-test. The results are shown in FIG. 1.

According to FIG. 1, it was confirmed that the expression level of the luciferase mRNA capped with Compound 12 was significantly superior, being approximately 2.3 times higher than that of the luciferase mRNA capped with $^{7m}GpppA_{(2'OMe)}pU$.

**Experimental Example 3: In Vivo Expression Test of Luciferase mRNA Capped with Compound 12**

To confirm the effects of the capped luciferase mRNA-LNP complexes prepared in Experimental Example 2 in vivo, each mRNA-LNP complex was intravenously injected into the tails of mice (8-10 weeks old, CD-1, female).

Subsequently, after anesthetizing the mice with avertin (250 mg/kg), a substrate prepared by adding 1X PBS to a luciferase substrate stock solution was intravenously injected.

At 3, 6, 9, 24, 48, and 96 hours after administration, the mice were imaged using an IVIS (In Vivo Imaging System). The degree of luciferase expression was quantified as a Region of Interest (ROI) using Aura Imaging Software, and the total luciferase expression level was confirmed. Significance was verified using a t-test. The results are shown in FIG. 2.

According to the results in FIG. 2, it was confirmed that the luciferase mRNA capped with Compound 12 exhibited significantly superior expression, being approximately 2.8 times higher than that of the luciferase mRNA capped with $^{7m}GpppA_{(2'OMe)}pU$.

**Experimental Example 4: In Vitro Expression Test of Luciferase mRNA Capped with Compound 16**

In the same manner as Experimental Example 2, luciferase mRNA capped with Compound 16 prepared in Experimental Example 1 (prepared by adding UTP or $^{1m}\Psi TP$ during the preparation in Experimental Example 1) was encapsulated into LNPs, and the luciferase expression of the mRNA-LNP complex was confirmed in HEK293S cells. As a control, a luciferase mRNA-LNP complex capped with $^{7m}G_{(3'OMe)}pppA_{(2'OMe)}pG$ was prepared and used.

The luciferase mRNA-LNP complexes capped with Compound 16 or $^{7m}G_{(3'OMe)}pppA_{(2'OMe)}pG$ prepared in the above step were transfected into HEK293S cells. Specifically, HEK293S cells were seeded in 12-well plates at a density of

$1.0 \times 10^6$ cells per well. The cells were treated with 3 μg of the luciferase mRNA-LNP complexes capped with Compound 16 or $^{7m}G_{(3'OMe)}pppA_{(2'OMe)}pG$, mixed using a pipette, and then incubated overnight. To confirm luciferase expression, $0.05 \times 10^6$ cells were harvested from each well at 24, 48, and 72 hours post-transfection. Luciferase expression was measured using the Steady-Glo Luciferase Assay System (Promega, #E2520) according to the manufacturer's manual. The results are shown in FIG. 3.

According to the results in FIG. 3, it was confirmed that the luciferase mRNA capped with Compound 16 exhibited significantly superior expression compared to the luciferase mRNA capped with $^{7m}G_{(3'OMe)}pppA_{(2'OMe)}pG$ at all time points, regardless of the type of UTP used during mRNA preparation. In particular, it was confirmed that the luciferase expression was highest in mRNA prepared with $^{1m}\Psi TP$ (N1-MethylPseudouridine 5'-Triphosphate).

**Experimental Example 5: In Vivo Expression Test of Luciferase mRNA Capped with Compound 16**

To confirm the effects of the capped luciferase mRNA-LNP complexes prepared in Experimental Example 4 *in vivo,* each mRNA-LNP complex was intravenously injected into the tails of mice (8-10 weeks old, CD-1, female).

Subsequently, after anesthetizing the mice with avertin (250 mg/kg), a substrate prepared by adding 1X PBS to a luciferase substrate stock solution was intravenously injected.

At 3, 6, 9, 24, 48, and 96 hours after administration, the mice were imaged using an IVIS, and the degree of luciferase expression (Region of Interest, ROI) was quantified using Aura Imaging Software. The results are shown in Table 3 below and illustrated in FIG. 4 and 5.

[Table 3]

| | NC | $^{7m}G_{(3'oMe)}pppA_{(2'OMe)}pG$ UTP | $^{7m}G_{(3'OMe)}pppA_{(2'OMe)}pG$ $^{1m}\Psi TP$ | Compound 16 UTP | Compound 16 $^{1m}\Psi TP$ |
|---|---|---|---|---|---|
| **3hr** | 2.26E+05 | 2.70E+10 | 3.85E+10 | 7.73E+10 | 6.67E+10 |
| **6hr** | 2.40E+05 | 1.50E+10 | 2.02E+10 | 3.45E+10 | 3.40E+10 |
| **9hr** | 2.79E+05 | 4.68E+09 | 8.71E+09 | 1.23E+10 | 1.69E+10 |
| **24hr** | 2.69E+05 | 2.22E+08 | 7.46E+08 | 8.56E+08 | 7.55E+08 |
| **48hr** | 2.81E+05 | 2.77E+07 | 7.62E+07 | 8.66E+07 | 7.69E+07 |
| **96hr** | 2.56E+05 | 2.43E+06 | 9.02E+06 | 7.83E+06 | 1.00E+07 |

According to Table 2, FIG. 4, and FIG. 5, it was confirmed that the expression of luciferase mRNA prepared with Compound 16 and UTP was approximately 2.8 times higher 3 hours post-injection compared to that of luciferase mRNA prepared with $^{7m}G_{(3'OMe)}pppA_{(2'OMe)}pG$ and UTP. Additionally, the expression of luciferase mRNA prepared with Compound 16 and $^{1m}\Psi TP$ was confirmed to be approximately 1.73 times higher than that of luciferase mRNA prepared with $^{7m}G_{(3'OMe)}pppA_{(2'OMe)}pG$ and $^{1m}\Psi TP$. Furthermore, the total luciferase expression level was highest in the luciferase mRNA prepared with Compound 16 and $^{1m}\Psi TP$.

Therefore, it was confirmed that Compound 16 exhibited significantly superior luciferase expression compared to $^{7m}G_{(3'OMe)}pppA_{(2'OMe)}pG$, regardless of the type of UTP used.

**Experimental Example 6: In Vitro Expression Test of Luciferase mRNA Capped with Compound 18**

The expression levels of luciferase mRNA capped with Compound 18 or $^{7m}GpppA_{(2'OMe)}pU$, prepared in Experimental Example 1, were confirmed in HEK293T cells.

HEK293T cells were seeded in 12-well plates at a density of $2.5 \times 10^5$ cells per well 24 hours prior to transfection, and transfection was performed using Lipofectamine™ MessengerMAX™ Transfection Reagent (ThermoFisher, #LMRNA001) according to the manufacturer's manual. Specifically, Solution A was prepared by diluting 3L of Lipofectamine™ MessengerMAX™ in 50 μL of Opti-MEM and reacting it at room temperature for 10 minutes, and Solution B was prepared by diluting 1.5 μg of the luciferase mRNA in 50 μL of Opti-MEM. After mixing Solution A and Solution B and reacting them at room temperature for 5 minutes, 100 μL of the mixed solution per well was treated to perform transfection.

Cells were harvested at 4, 8, 12, 24, and 48 hours post-transfection, and luciferase expression was measured using the Luciferase Assay System (Promega, #E1501) according to the manufacturer's manual, after which the total luciferase expression level was confirmed. The results are shown in FIG. 6.

According to FIG. 6, it was confirmed that the luciferase mRNA capped with Compound 18 exhibited an expression level equivalent to that of the luciferase mRNA capped with $^{7m}GpppA_{(2'OMe)}pU$.

## Claims

**1.** A compound of Chemical Formula 1 below or a pharmaceutically acceptable salt thereof:

[Chemical Formula 1]

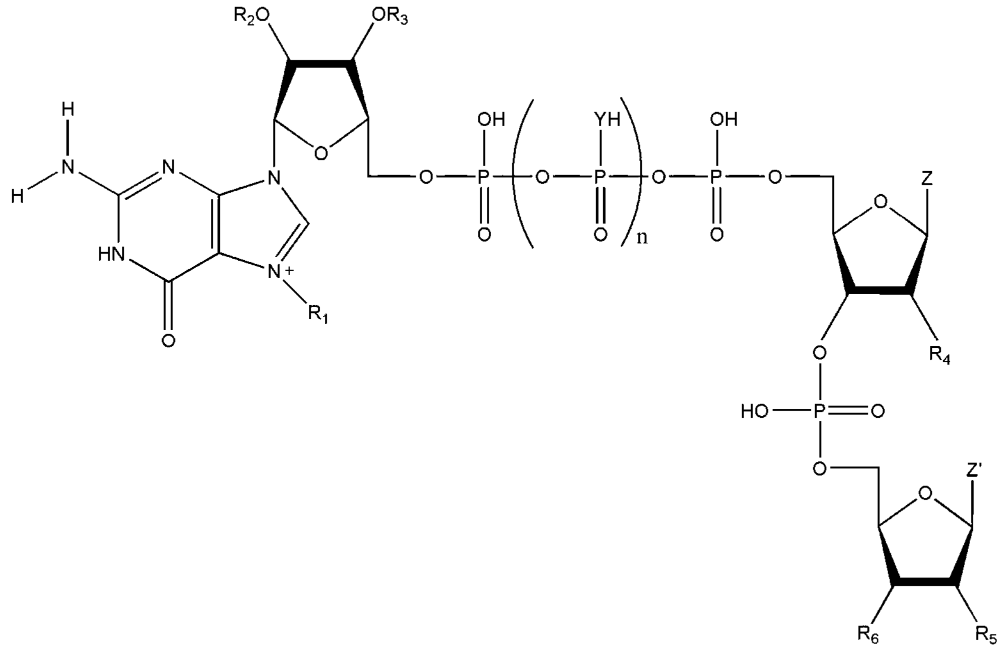

wherein

n = 0, 1, or 2;
YH is OH or SH;
$R_1$ is $C_{1-6}$ alkyl or $CH_2Ph$;
$R_2$ and $R_3$ are each independently H or a sulfonyl-containing group, wherein at least one of $R_2$ and $R_3$ is a sulfonyl-containing group, and the sulfonyl-containing group is independently selected from the group consisting of mesyl, esyl, triflyl, tresyl, tosyl, brosyl, nosyl, and dansyl groups;
$R_4$ and $R_5$ are each independently OH or methoxy;
$R_6$ is OH or a mononucleotide or oligonucleotide having 1 to 7 bases;
Z is each independently a natural, modified or unnatural nucleoside base; and
Z' is each independently

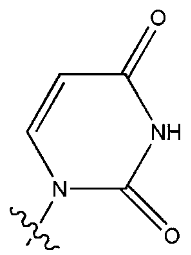,

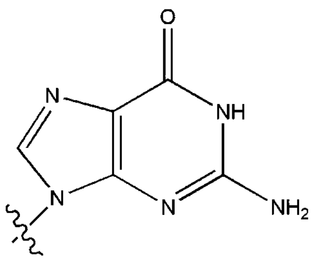,

or

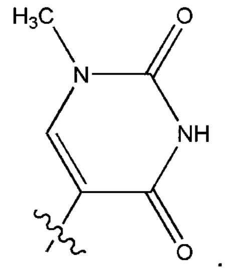

2. The compound of claim 1, wherein the compound has a structure of Chemical Formula 1a below:

[Chemical Formula 1a]

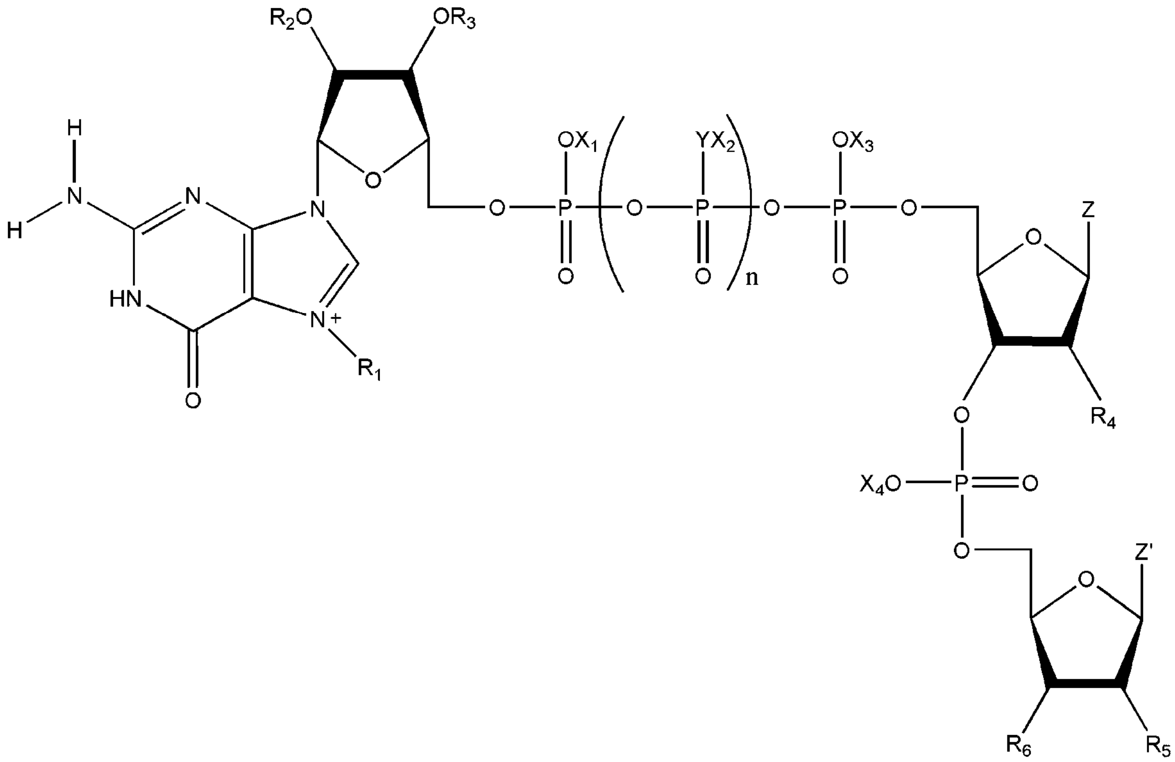

wherein

n = 0, 1, or 2;
$X_1$, $X_2$, $X_3$, and $X_4$ are each independently absent, or a monovalent metal ion, a divalent metal ion, or a combination thereof, wherein $X_1$, $X_2$, $X_3$, and $X_4$ are selected to electrically neutralize the compound of Chemical Formula 1;
Y is O or S;
$R_1$ is $C_{1-6}$ alkyl or $CH_2Ph$;
$R_2$ and $R_3$ are each independently H or a sulfonyl-containing group, wherein at least one of $R_2$ and $R_3$ is a sulfonyl-containing group, and the sulfonyl-containing group is independently selected from the group consisting of mesyl, esyl, triflyl, tresyl, tosyl, brosyl, nosyl, and dansyl groups;
$R_4$ and $R_5$ are each independently OH or methoxy;
$R_6$ is OH or a mononucleotide or oligonucleotide having 1 to 7 bases;
Z is each independently a natural, modified, or unnatural nucleoside base; and
Z' is each independently

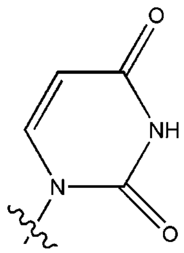,
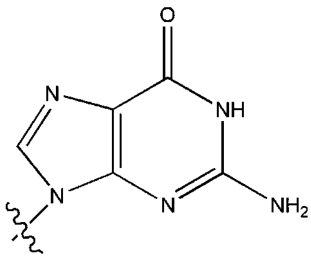,

or

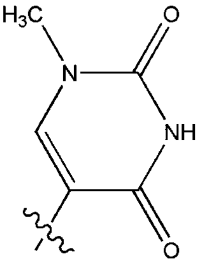 .

.

3. The compound of claim 2, wherein the monovalent metal ion is selected from the group consisting of $Na^+$, $Li^+$, and $K^+$, and the divalent metal ion is selected from the group consisting of $Mg^{2+}$, $Zn^{2+}$, and $Ca^{2+}$.

4. The compound of claim 2, wherein Z is each independently selected from the group consisting of guanine, adenine, cytosine, thymine, uracil, and modified forms thereof.

5. The compound of claim 2, wherein

n = 0, 1, or 2;
$X_1$, $X_2$, $X_3$, and $X_4$ are each independently absent or Na;
Y is O or S;
$R_1$ is $C_{1-6}$ alkyl;
$R_2$ and $R_3$ are each independently H or a sulfonyl-containing group, wherein at least one of $R_2$ and $R_3$ is a sulfonyl-containing group, and the sulfonyl-containing group is independently selected from the group consisting of mesyl, esyl, and tosyl groups;
$R_4$ and $R_5$ are each independently OH or methoxy;
$R_6$ is OH or a mononucleotide or oligonucleotide having 1 to 3 bases;
Z is each independently a natural, modified, or unnatural nucleoside base; and
Z' is each independently

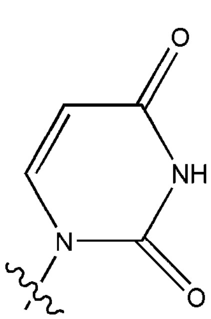, 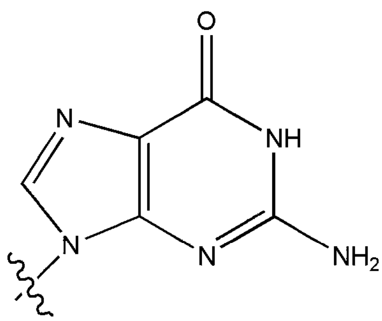, or 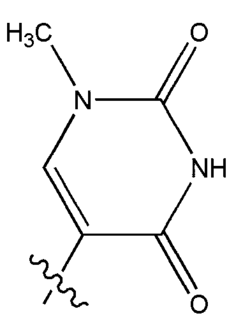

6. The compound of claim 1, wherein the compound is selected from the group consisting of the compounds represented by chemical formulae below, or a pharmaceutically acceptable salt thereof:

$^{7m}G_{(3'OMs)}pppA_{(2'OMe)}pU$

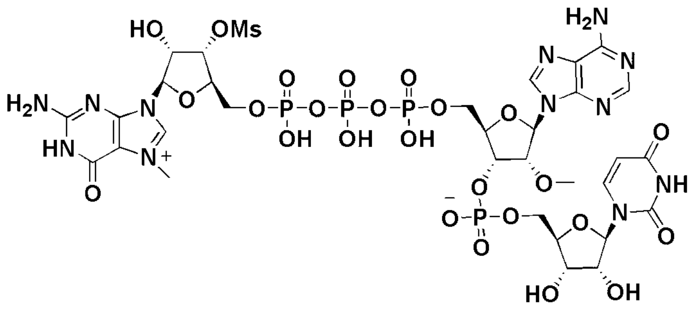

;

$^{7m}G_{(3'OMs)}pp_{s}pA_{(2'OMe)}pG$

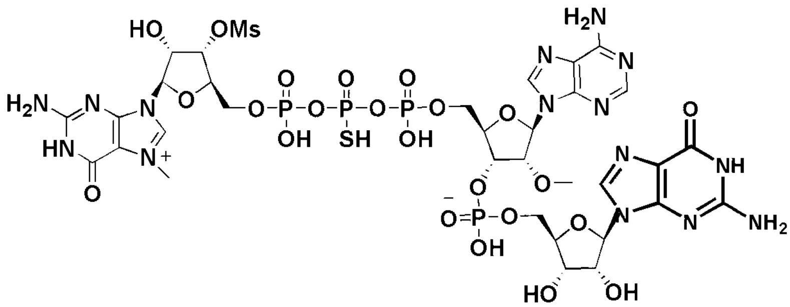

;

and

$^{7m}G_{(3'OMs)}pppA_{(2'OMe)}p^{1m\Psi}$

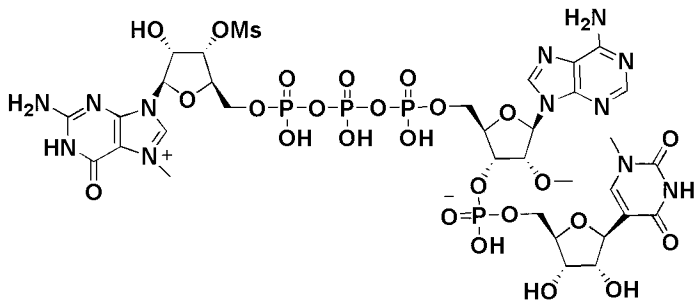

.

**7.** An mRNA 5'-capped with a cap analog that is the compound according to any one of claims 1 to 6.

**8.** A method for preparing 5'-capped mRNA, the method comprising adding a cap analog which is the compound according to any one of claims 1 to 6, during synthesis of the mRNA.

**9.** A composition or kit for preparing 5'-capped mRNA, comprising a cap analog that is the compound according to any one of claims 1 to 6.

**10.** A pharmaceutical composition for expressing a target peptide or protein, comprising: mRNA 5'-capped with a cap analog that is the compound according to any one of claims 1 to 6; and a pharmaceutically acceptable carrier.

**11.** A cell comprising the 5'-capped mRNA of claim 7.

**12.** A cell comprising a protein or peptide translated from the 5'-capped mRNA of claim 7.

FIG. 1

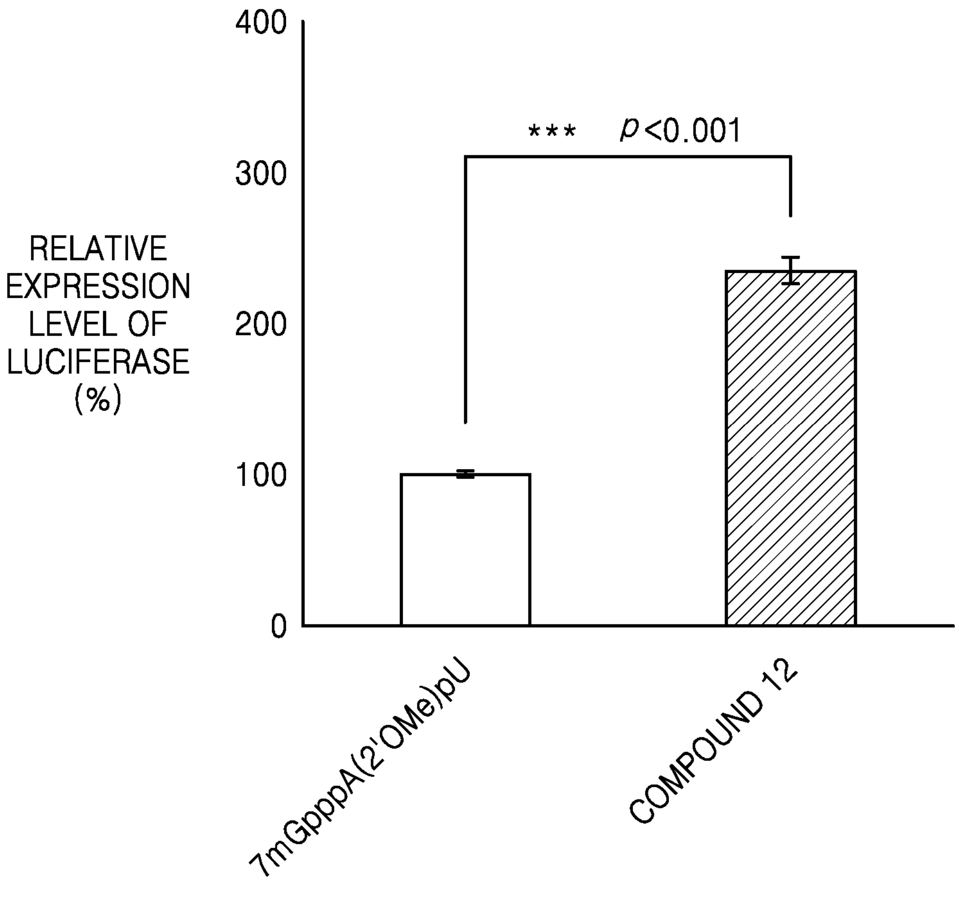
400
300
200
100
0
RELATIVE EXPRESSION LEVEL OF LUCIFERASE (%)
*** p<0.001
7mGpppA(2'OMe)pU
COMPOUND 12

FIG. 2

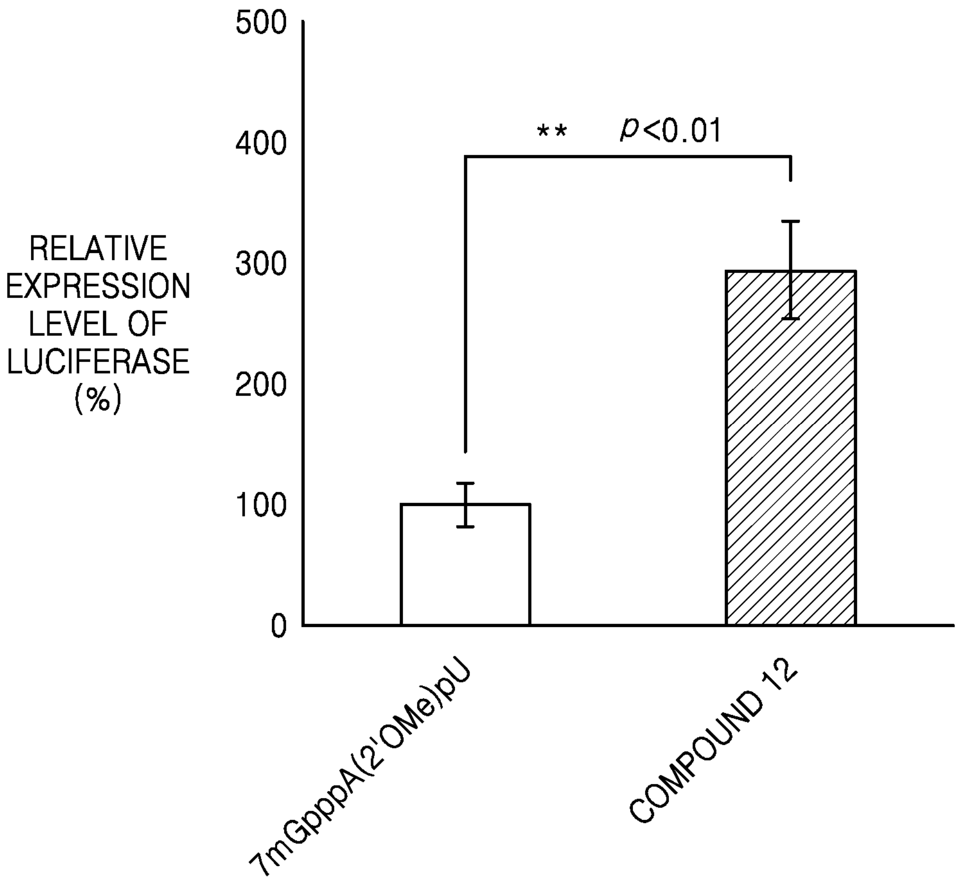

RELATIVE EXPRESSION LEVEL OF LUCIFERASE (%)
500
400
300
200
100
0
** p<0.01
7mGpppA(2'OMe)pU
COMPOUND 12

# FIG. 3

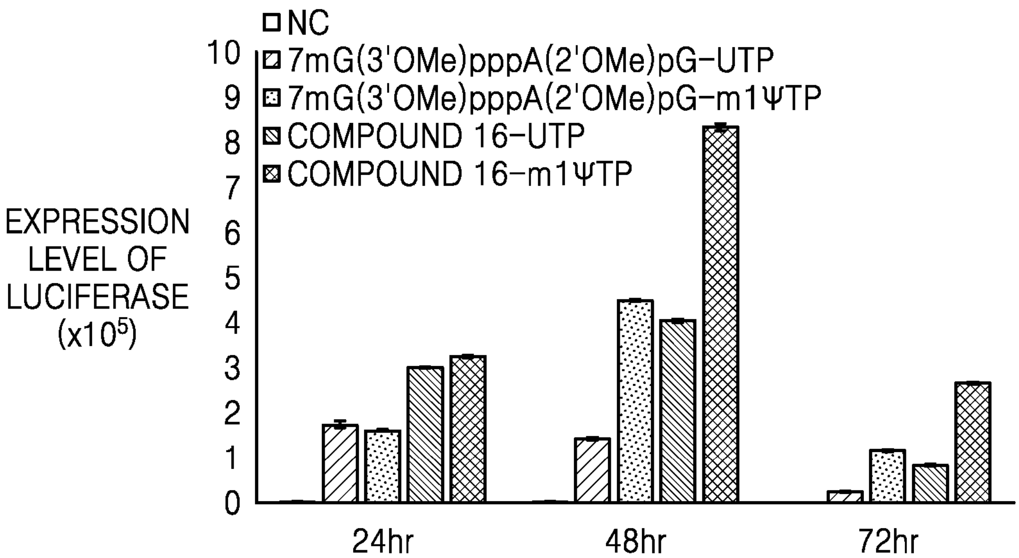
NC
7mG(3'OMe)pppA(2'OMe)pG-UTP
7mG(3'OMe)pppA(2'OMe)pG-m1ΨTP
COMPOUND 16-UTP
COMPOUND 16-m1ΨTP
EXPRESSION LEVEL OF LUCIFERASE ($x10^5$)
0
1
2
3
4
5
6
7
8
9
10
24hr
48hr
72hr

FIG. 4

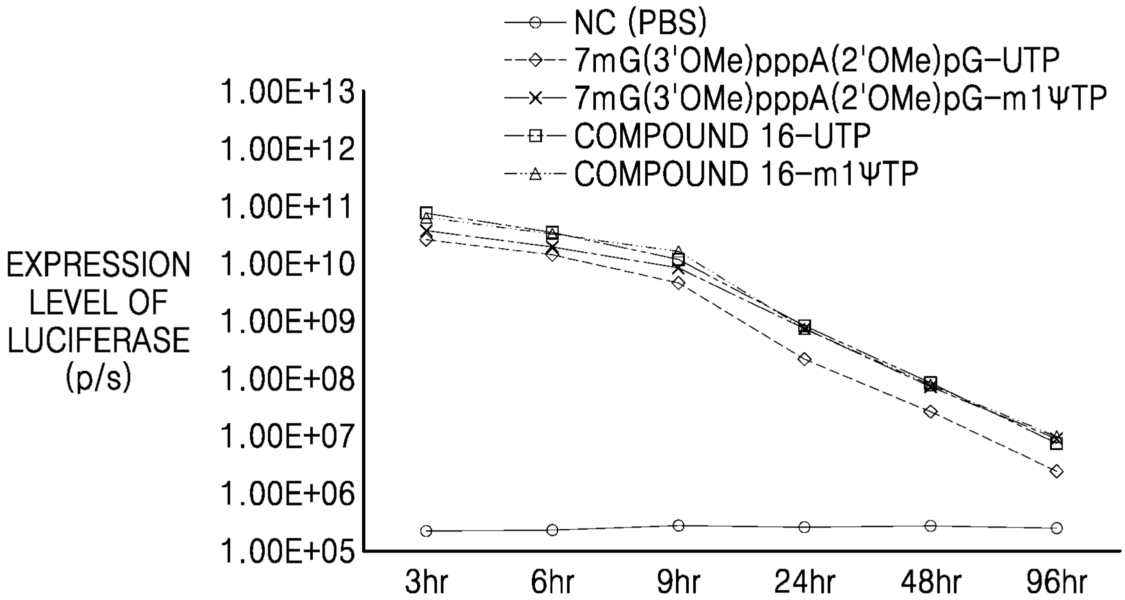
NC (PBS)
7mG(3'OMe)pppA(2'OMe)pG-UTP
7mG(3'OMe)pppA(2'OMe)pG-m1ΨTP
COMPOUND 16-UTP
COMPOUND 16-m1ΨTP
EXPRESSION LEVEL OF LUCIFERASE (p/s)
1.00E+13
1.00E+12
1.00E+11
1.00E+10
1.00E+09
1.00E+08
1.00E+07
1.00E+06
1.00E+05
3hr
6hr
9hr
24hr
48hr
96hr

FIG. 5

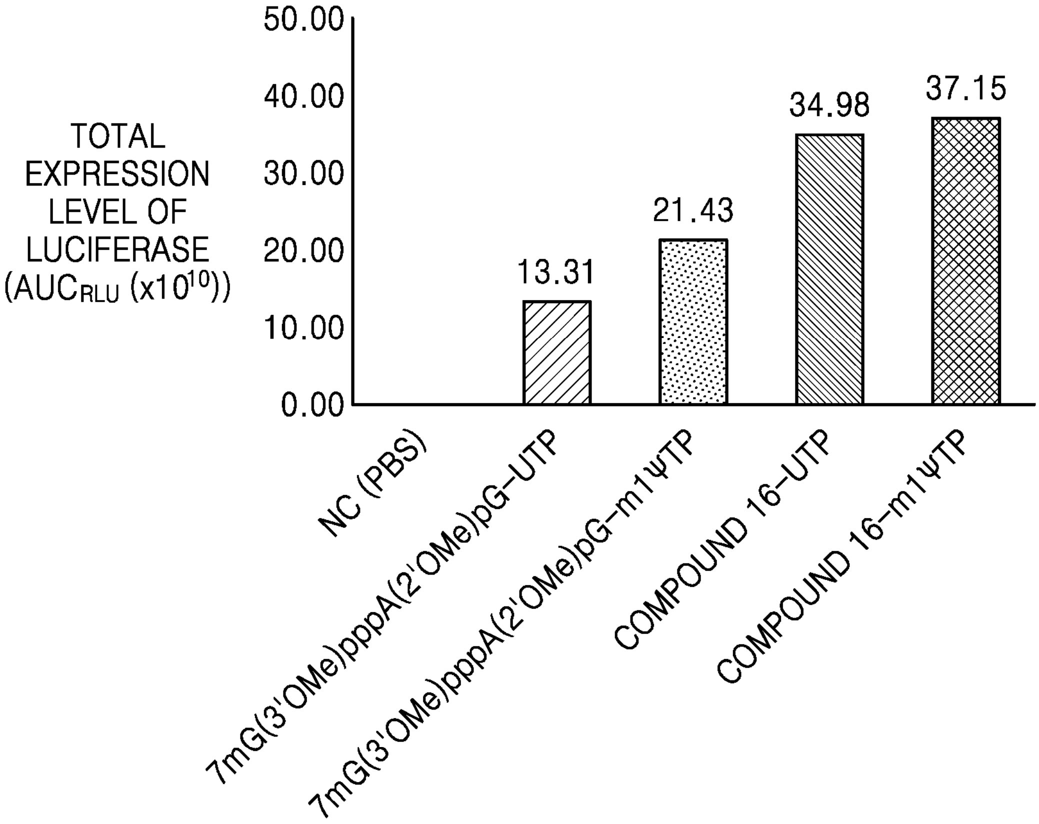
TOTAL EXPRESSION LEVEL OF LUCIFERASE (AUCRLU (x10¹⁰))
50.00
40.00
30.00
20.00
10.00
0.00
13.31
21.43
34.98
37.15
NC (PBS)
7mG(3'OMe)pppA(2'OMe)pG-UTP
7mG(3'OMe)pppA(2'OMe)pG-m1ΨTP
COMPOUND 16-UTP
COMPOUND 16-m1ΨTP

FIG. 6

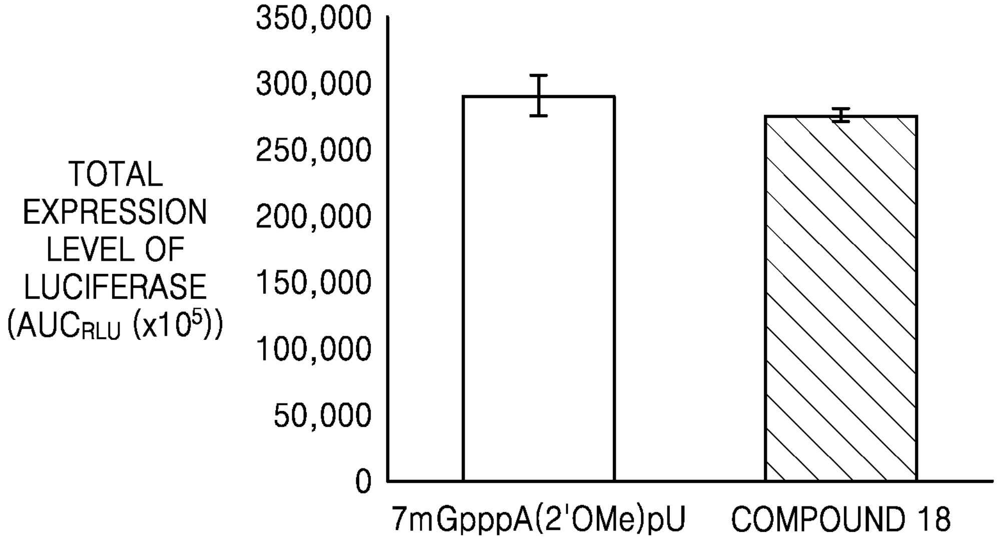
350,000
300,000
250,000
200,000
150,000
100,000
50,000
0
TOTAL EXPRESSION LEVEL OF LUCIFERASE (AUCRLU (x10^5))
7mGpppA(2'OMe)pU
COMPOUND 18

# INTERNATIONAL SEARCH REPORT

International application No.
**PCT/KR2024/014788**

**A. CLASSIFICATION OF SUBJECT MATTER**

**C07H 21/02**(2006.01)i; **C07H 1/04**(2006.01)i; **A61K 39/00**(2006.01)i; **A61K 48/00**(2006.01)i; **C12N 15/63**(2006.01)i

According to International Patent Classification (IPC) or to both national classification and IPC

**B. FIELDS SEARCHED**

Minimum documentation searched (classification system followed by classification symbols)

C07H 21/02(2006.01); A61K 39/00(2006.01); C07H 1/00(2006.01); C12N 15/113(2010.01); C12P 19/34(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 5'캡 유사체(5'cap analogue), mRNA, 뉴클레오티드(nucleotide), 설포닐-함유 기(sulfonyl-containing group), 뉴클레오시드 염기(nucleoside base)

**C. DOCUMENTS CONSIDERED TO BE RELEVANT**

| Category* | Citation of document, with indication, where appropriate, of the relevant passages | Relevant to claim No. |
|---|---|---|
| X | US 2014-0147454 A1 (CHAKRABORTY, T. et al.) 29 May 2014 (2014-05-29)<br>See claims 1, 2, 18 and 19; paragraphs [0003], [0008], [0009], [0157], [0233], [0255], [0256], [0368], [0373], [0465], [0466], [0469] and [0640]; pages 31 and 33; and table 1. | 1-12 |
| A | CN 114540444 A (JIANGSU SHENKI BIOTECHNOLOGY CO., LTD.) 27 May 2022 (2022-05-27)<br>See claims 1, 2 and 4-6; and figures 1-4. | 1-12 |
| A | CN 113957108 A (HONGENE BIOTECH CORPORATION et al.) 21 January 2022 (2022-01-21)<br>See claims 1-4 and 10; and paragraphs [0118]-[0140]. | 1-12 |
| A | KR 10-2366490 B1 (ST PHARM CO., LTD.) 23 February 2022 (2022-02-23)<br>See claims 1-3, 6, 12, 15 and 17. | 1-12 |
| A | WO 2023-025073 A1 (SHANGHAI HONGENE BIOTECH CORPORATION et al.) 02 March 2023 (2023-03-02)<br>See claims 1, 13, 16 and 20. | 1-12 |

☐ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 January 2025** | **09 January 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2024/014788**

| Patent document cited in search report | Publication date (day/month/year) | Patent family member(s) | Publication date (day/month/year) |
|---|---|---|---|
| US 2014-0147454 A1 | 29 May 2014 | AU 2012-318752 A1 | 24 April 2014 |
| | | AU 2012-318752 B2 | 31 August 2017 |
| | | AU 2012-352180 A1 | 31 July 2014 |
| | | AU 2013-243834 A1 | 30 October 2014 |
| | | AU 2013-243946 A1 | 30 October 2014 |
| | | AU 2013-243947 A1 | 30 October 2014 |
| | | AU 2013-243948 A1 | 30 October 2014 |
| | | AU 2013-243949 A1 | 30 October 2014 |
| | | AU 2013-243950 A1 | 30 October 2014 |
| | | AU 2013-243951 A1 | 30 October 2014 |
| | | AU 2013-243952 A1 | 30 October 2014 |
| | | AU 2013-243953 A1 | 30 October 2014 |
| | | AU 2013-243954 A1 | 30 October 2014 |
| | | AU 2013-243955 A1 | 30 October 2014 |
| | | AU 2013-243955 B2 | 22 February 2018 |
| | | AU 2013-348363 A1 | 11 June 2015 |
| | | AU 2016-231503 A1 | 06 October 2016 |
| | | AU 2017-202228 A1 | 27 April 2017 |
| | | AU 2017-202228 B2 | 14 March 2019 |
| | | AU 2017-213503 A1 | 31 August 2017 |
| | | AU 2017-213503 B2 | 20 June 2019 |
| | | AU 2017-232121 A1 | 12 October 2017 |
| | | AU 2017-232121 B2 | 09 May 2019 |
| | | AU 2018-200373 A1 | 22 March 2018 |
| | | AU 2018-200374 A1 | 22 March 2018 |
| | | AU 2018-200374 B2 | 19 November 2020 |
| | | AU 2018-200375 A1 | 22 March 2018 |
| | | AU 2018-200375 B2 | 19 November 2020 |
| | | AU 2018-200377 A1 | 22 March 2018 |
| | | AU 2018-200379 A1 | 22 March 2018 |
| | | AU 2018-200380 A1 | 22 March 2018 |
| | | AU 2018-200381 A1 | 01 February 2018 |
| | | AU 2018-207584 A1 | 09 August 2018 |
| | | AU 2018-247318 A1 | 08 November 2018 |
| | | AU 2018-260928 A1 | 06 December 2018 |
| | | AU 2019-202835 A1 | 16 May 2019 |
| | | AU 2019-203876 A1 | 20 June 2019 |
| | | AU 2019-219843 A1 | 12 September 2019 |
| | | AU 2020-257150 A1 | 19 November 2020 |
| | | AU 2021-200507 A1 | 01 April 2021 |
| | | AU 2021-201508 A1 | 01 April 2021 |
| | | AU 2021-202758 A1 | 27 May 2021 |
| | | AU 2023-200127 A1 | 16 February 2023 |
| | | AU 2023-214237 A1 | 31 August 2023 |
| | | AU 2023-263451 A1 | 30 November 2023 |
| | | BR 112014007852 A2 | 18 April 2017 |
| | | BR 112014007852 B1 | 03 November 2021 |
| | | CA 2850624 A1 | 11 April 2013 |
| | | CA 2859387 A1 | 20 June 2013 |
| | | CA 2866919 A1 | 03 October 2013 |

Form PCT/ISA/210 (patent family annex) (July 2022)

**INTERNATIONAL SEARCH REPORT**
**Information on patent family members**

International application No.
**PCT/KR2024/014788**

| Patent document cited in search report | Publication date (day/month/year) | Patent family member(s) | Publication date (day/month/year) |
|---|---|---|---|
| | | CA 2866919 C | 21 August 2018 |
| | | CA 2868391 A1 | 10 October 2013 |
| | | CA 2868393 A1 | 10 October 2013 |
| | | CA 2868398 A1 | 10 October 2013 |
| | | CA 2868418 A1 | 10 October 2013 |
| | | CA 2868422 A1 | 10 October 2013 |
| | | CA 2868429 A1 | 10 October 2013 |
| | | CA 2868434 A1 | 10 October 2013 |
| | | CA 2868438 A1 | 10 October 2013 |
| | | CA 2868440 A1 | 10 October 2013 |
| | | CA 2868996 A1 | 10 October 2013 |
| | | CA 2869005 A1 | 10 October 2013 |
| | | CA 2892529 A1 | 30 May 2014 |
| | | CA 2892529 C | 25 April 2023 |
| | | CA 2894462 A1 | 19 June 2014 |
| | | CA 3018046 A1 | 20 June 2013 |
| | | CN 103974724 A | 06 August 2014 |
| | | CN 103974724 B | 30 August 2019 |
| | | CN 104114572 A | 22 October 2014 |
| | | CN 104411338 A | 11 March 2015 |
| | | CN 104870022 A | 26 August 2015 |
| | | CN 108949772 A | 07 December 2018 |
| | | CN 110201187 A | 06 September 2019 |
| | | CN 110511939 A | 29 November 2019 |
| | | CN 112390871 A | 23 February 2021 |
| | | CY 1125029 T1 | 24 March 2023 |
| | | EP 2763701 A1 | 13 August 2014 |
| | | EP 2763701 B1 | 19 December 2018 |
| | | EP 2791160 A1 | 22 October 2014 |
| | | EP 2791160 B1 | 02 March 2022 |
| | | EP 2833892 A2 | 11 February 2015 |
| | | EP 2833894 A1 | 11 February 2015 |
| | | EP 2833920 A2 | 11 February 2015 |
| | | EP 2833921 A2 | 11 February 2015 |
| | | EP 2833922 A2 | 11 February 2015 |
| | | EP 2833922 B1 | 05 December 2018 |
| | | EP 2833923 A1 | 11 February 2015 |
| | | EP 2834259 A1 | 11 February 2015 |
| | | EP 2834260 A1 | 11 February 2015 |
| | | EP 2834358 A2 | 11 February 2015 |
| | | EP 2847329 A1 | 18 March 2015 |
| | | EP 2849799 A2 | 25 March 2015 |
| | | EP 2922554 A1 | 30 September 2015 |
| | | EP 2922554 B1 | 23 February 2022 |
| | | EP 2931319 A1 | 21 October 2015 |
| | | EP 2931319 A4 | 04 May 2016 |
| | | EP 2931319 B1 | 21 August 2019 |
| | | EP 2964234 A1 | 13 January 2016 |
| | | EP 3492109 A1 | 05 June 2019 |
| | | EP 3492109 B1 | 04 March 2020 |

Form PCT/ISA/210 (patent family annex) (July 2022)

**INTERNATIONAL SEARCH REPORT**
**Information on patent family members**

International application No.
**PCT/KR2024/014788**

| Patent document cited in search report | Publication date (day/month/year) | Patent family member(s) | | | Publication date (day/month/year) |
|---|---|---|---|---|---|
| | | EP | 3501550 | A1 | 26 June 2019 |
| | | EP | 3505176 | A1 | 03 July 2019 |
| | | EP | 3520820 | A1 | 07 August 2019 |
| | | EP | 3520821 | A1 | 07 August 2019 |
| | | EP | 3682905 | A1 | 22 July 2020 |
| | | EP | 3682905 | B1 | 01 December 2021 |
| | | EP | 3978030 | A1 | 06 April 2022 |
| | | EP | 4015005 | A1 | 22 June 2022 |
| | | EP | 4074834 | A1 | 19 October 2022 |
| | | EP | 4144378 | A1 | 08 March 2023 |
| | | ES | 2911677 | T3 | 20 May 2022 |
| | | ES | 2921623 | T3 | 30 August 2022 |
| | | ES | 2923757 | T3 | 30 September 2022 |
| | | JP | 2014-530601 | A | 20 November 2014 |
| | | JP | 2015-501844 | A | 19 January 2015 |
| | | JP | 2015-513912 | A | 18 May 2015 |
| | | JP | 2015-513913 | A | 18 May 2015 |
| | | JP | 2015-513914 | A | 18 May 2015 |
| | | JP | 2015-513916 | A | 18 May 2015 |
| | | JP | 2015-516143 | A | 08 June 2015 |
| | | JP | 2015-517995 | A | 25 June 2015 |
| | | JP | 2015-518704 | A | 06 July 2015 |
| | | JP | 2015-518705 | A | 06 July 2015 |
| | | JP | 2015-518816 | A | 06 July 2015 |
| | | JP | 2015-519040 | A | 09 July 2015 |
| | | JP | 2015-519881 | A | 16 July 2015 |
| | | JP | 2015-535430 | A | 14 December 2015 |
| | | JP | 2017-113029 | A | 29 June 2017 |
| | | JP | 2017-121239 | A | 13 July 2017 |
| | | JP | 2017-121240 | A | 13 July 2017 |
| | | JP | 2017-121241 | A | 13 July 2017 |
| | | JP | 2017-121243 | A | 13 July 2017 |
| | | JP | 2017-121244 | A | 13 July 2017 |
| | | JP | 2017-123847 | A | 20 July 2017 |
| | | JP | 2017-123853 | A | 20 July 2017 |
| | | JP | 2017-140048 | A | 17 August 2017 |
| | | JP | 2017-141230 | A | 17 August 2017 |
| | | JP | 2017-197545 | A | 02 November 2017 |
| | | JP | 2018-023373 | A | 15 February 2018 |
| | | JP | 2018-164458 | A | 25 October 2018 |
| | | JP | 2018-166528 | A | 01 November 2018 |
| | | JP | 2019-030327 | A | 28 February 2019 |
| | | JP | 2019-033757 | A | 07 March 2019 |
| | | JP | 2019-054813 | A | 11 April 2019 |
| | | JP | 2019-059793 | A | 18 April 2019 |
| | | JP | 2019-107022 | A | 04 July 2019 |
| | | JP | 2019-208511 | A | 12 December 2019 |
| | | JP | 2019-216733 | A | 26 December 2019 |
| | | JP | 2020-072670 | A | 14 May 2020 |
| | | JP | 2020-072766 | A | 14 May 2020 |

Form PCT/ISA/210 (patent family annex) (July 2022)

**INTERNATIONAL SEARCH REPORT**
**Information on patent family members**

International application No.
**PCT/KR2024/014788**

| Patent document cited in search report | Publication date (day/month/year) | Patent family member(s) | Publication date (day/month/year) |
|---|---|---|---|
| | | JP 2020-158508 A | 01 October 2020 |
| | | JP 2021-045163 A | 25 March 2021 |
| | | JP 2021-192606 A | 23 December 2021 |
| | | JP 2022-023036 A | 07 February 2022 |
| | | JP 2022-036938 A | 08 March 2022 |
| | | JP 2022-060269 A | 14 April 2022 |
| | | JP 2022-078081 A | 24 May 2022 |
| | | JP 2022-093332 A | 23 June 2022 |
| | | JP 2022-122923 A | 23 August 2022 |
| | | JP 2023-021215 A | 10 February 2023 |
| | | JP 2023-130471 A | 20 September 2023 |
| | | JP 2023-145599 A | 11 October 2023 |
| | | JP 2023-175888 A | 12 December 2023 |
| | | JP 2024-036694 A | 15 March 2024 |
| | | JP 2024-063025 A | 10 May 2024 |
| | | JP 2024-123008 A | 10 September 2024 |
| | | JP 6113737 B2 | 12 April 2017 |
| | | JP 6144355 B2 | 07 June 2017 |
| | | JP 6189415 B2 | 30 August 2017 |
| | | JP 6348482 B2 | 27 June 2018 |
| | | JP 6377804 B2 | 22 August 2018 |
| | | JP 6388977 B2 | 12 September 2018 |
| | | JP 6424156 B2 | 14 November 2018 |
| | | JP 6426217 B2 | 21 November 2018 |
| | | JP 6430552 B2 | 28 November 2018 |
| | | JP 6449356 B2 | 09 January 2019 |
| | | JP 6553104 B2 | 31 July 2019 |
| | | JP 6574032 B2 | 11 September 2019 |
| | | JP 6666391 B2 | 13 March 2020 |
| | | JP 6921797 B2 | 18 August 2021 |
| | | JP 6946384 B2 | 06 October 2021 |
| | | JP 6953135 B2 | 27 October 2021 |
| | | JP 6971953 B2 | 24 November 2021 |
| | | JP 7019639 B2 | 15 February 2022 |
| | | JP 7047002 B2 | 04 April 2022 |
| | | KR 10-2014-0068245 A | 05 June 2014 |
| | | KR 10-2014-0102759 A | 22 August 2014 |
| | | KR 10-2014061 B1 | 28 August 2019 |
| | | KR 10-2019-0099538 A | 27 August 2019 |
| CN 114540444 A | 27 May 2022 | CN 114540444 B | 09 August 2022 |
| | | WO 2023-202199 A1 | 26 October 2023 |
| | | WO 2023-202199 A9 | 21 November 2024 |
| CN 113957108 A | 21 January 2022 | None | |
| KR 10-2366490 B1 | 23 February 2022 | CA 3198727 A1 | 28 April 2022 |
| | | CN 116438306 A | 14 July 2023 |
| | | EP 4234567 A1 | 30 August 2023 |
| | | IL 302215 A | 01 June 2023 |
| | | JP 2023-549592 A | 28 November 2023 |
| | | US 2023-0382943 A1 | 30 November 2023 |
| | | WO 2022-086140 A1 | 28 April 2022 |

Form PCT/ISA/210 (patent family annex) (July 2022)

**INTERNATIONAL SEARCH REPORT**
**Information on patent family members**

International application No.
**PCT/KR2024/014788**

| Patent document cited in search report | Publication date (day/month/year) | Patent family member(s) | Publication date (day/month/year) |
|---|---|---|---|
| WO 2023-025073 A1 | 02 March 2023 | CN 113603739 A | 05 November 2021 |
| | | CN 113603739 B | 23 August 2024 |
| | | EP 4393936 A1 | 03 July 2024 |
| | | JP 2024-534261 A | 18 September 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10913768 B
- US 7074596 B
- US 6762298 B